# EUROPÄISCHE PATENTSCHRIFT

(11) **EP 4 311 510 B1**
(45) Veröffentlichungstag und Bekanntmachung des Hinweises auf die Patenterteilung: **05.11.2025**
(21) Anmeldenummer: 23188209.3
(22) Anmeldetag: 27.07.2023
(51) Int. Cl.: A61B 17/92, A61B 17/84

(54) **MEDIZINISCHES SCHLAGINSTRUMENT**
MEDICAL PERCUSSION INSTRUMENT
INSTRUMENT MÉDICAL À PERCUSSION

(30) Priorität: 28.07.2022 DE 102022118986
(43) Veröffentlichungstag der Anmeldung: 31.01.2024
(73) Patentinhaber: Aesculap AG, 78532 Tuttlingen (DE)
(72) Erfinder: Nonnenmann, Martin, 78573 Wurmlingen (DE); Buggle, Mattea, 74193 Schwaigern (DE)
(74) Vertreter: Hoeger, Stellrecht & Partner Patentanwälte mbB

(56) Entgegenhaltungen:
- EP-A1- 0 780 090
- EP-A2- 1 308 143
- JP-A- 2016 022 234
- US-A1- 2006 178 673
- US-A1- 2018 177 536
- US-A1- 2023 139 058

## Beschreibung

Die vorliegende Erfindung betrifft ein medizinisches Schlaginstrument, insbesondere in Form eines Schlaghammers, mit einem eine Schaftlängsrichtung sowie ein proximales und ein distales Ende definierenden Schaft und einem am Schaft angeordneten, eine Längsachse definierenden Schlagkörper, wobei der Schlagkörper in einer Schlagstellung am Schaft zwischen einem eine distale Anschlagposition des Schlagkörpers definierenden distalen Anschlag und einem eine proximale Anschlagposition definierenden proximalen Anschlag bewegbar, insbesondere verschiebbar, ist zum Übertragen eines Schlagimpulses auf das distale Ende des Schafts in distaler beziehungsweise proximaler Richtung, wobei das Schlaginstrument eine Feststelleinrichtung umfasst zum temporären Feststellen des Schlagkörpers am Schaft in mindestens einer, insbesondere beliebigen, Feststellposition zwischen der distalen Anschlagposition und der proximalen Anschlagposition, wobei die Feststelleinrichtung von einer Fixierstellung, in welcher der Schlagkörper in einer der beliebigen Feststellpositionen am Schaft festgelegt ist, in die Schlagstellung, in welcher der Schlagkörper und der Schaft relativ zueinander bewegbar sind, überführbar ist und umgekehrt.

Medizinische Schlaginstrumente der eingangs beschriebenen Art sind beispielsweise in Form von Schlaghämmern, auch als sogenannte "slap hammer" bezeichnet, in unterschiedlichen Varianten bekannt. Distale Enden der Schlaginstrumente können beispielsweise mit Implantaten gekoppelt werden, um diese durch Übertragen von Schlagimpulsen beispielsweise in einen Knochen hineinzuschlagen oder aus diesem herauszuschlagen. Ein solches Schlaginstrument kann optional, insbesondere mit seinem distalen Ende, auch mit einem medizinischen Objekt gekoppelt werden. Aufgrund der Beweglichkeit des Schlagkörpers und des Schafts relativ zueinander ist es für eine solche Kopplung erforderlich, das Schlaginstrument mit zwei Händen handzuhaben, nämlich den Schaft und den Schlagkörper jeweils mit einer Hand zu halten. Dies ist jedoch insbesondere bei einem medizinischem Eingriff in einem Operationssaal nachteilig, da eine Operationszeit stets so kurz wie möglich gehalten werden sollte und zudem eine einfache Handhabung dabei eingesetzter Instrumente gewünscht wird.

In der US 2006/0178673 A1 sind ein verriegelbarer Schiebehammer und eine Griffvorrichtung beschrieben. Die JP 2016 022234 A betrifft einen medizinischen Schraubendreher. Aus der EP 1 308 143 A2 ist ein Schlaghammer bekannt. Die EP 0 780 090 A1 offenbart ein Knieprothesen-Implantationssystem mit einem Universalhandgriff. In der US 2018/0177536 A1 sind Schraubinstrumente und zugehörige Verfahren beschrieben. Die US 2023/0139058 A1 offenbart ein orthopädisches chirurgisches Instrument für zwei Einsatzzwecke.

Es ist daher eine Aufgabe der vorliegenden Erfindung, ein medizinisches Schlaginstrument der eingangs beschriebenen Art so zu verbessern, dass es einfacher handhabbar ist.

Diese Aufgabe wird bei einem medizinischen Schlaginstrument der eingangs beschriebenen Art erfindungsgemäß dadurch gelöst, dass der Schlagkörper in einer Grundstellung relativ zum Schaft festgestellt ist und die mindestens eine Feststellposition einnimmt und dass die Feststelleinrichtung eine Rückstelleinrichtung umfasst zum automatischen Halten der Feststelleinrichtung in der Fixierstellung.

Ein medizinisches Schlaginstrument mit einer solchen Feststelleinrichtung ermöglicht es einem Anwender insbesondere, den Schlagkörper am Schaft in einer oder mehreren, insbesondere einer Mehrzahl, zudem beliebigen Feststellpositionen festzustellen beziehungsweise zu arretieren. Feststellen in diesem Sinn ist so zu verstehen, dass der Schlagkörper und der Schaft relativ zueinander in der Feststellposition unbewegbar sind. Man kann auch sagen, der Schaft ist in der Feststellposition unbeweglich am Schlagkörper gehalten. Dieses Feststellen des Schlagkörpers am Schaft vereinfacht die Handhabung des medizinischen Schlaginstruments. Ist der Schlagkörper am Schaft festgestellt, kann ein Anwender ohne Zuhilfenahme einer zweiten Hand mit lediglich einer Hand das Schlaginstrument handhaben und beispielsweise mit einem medizinischen Objekt, zum Beispiel einem Befestigungselement in Form eines Pins, koppeln. Mit anderen Worten ermöglicht die Feststelleinrichtung insbesondere eine einfache Einhandbedienung des medizinischen Schlaginstruments. Gemäß der Erfindung ist vorgesehen, dass die Feststelleinrichtung von einer Fixierstellung, in welcher der Schlagkörper in einer der beliebigen Feststellpositionen am Schaft festgelegt ist, in die Schlagstellung, in welcher der Schlagkörper und der Schaft relativ zueinander bewegbar sind, überführbar ist und umgekehrt. Diese Ausgestaltung ermöglicht es einem Anwender insbesondere, den Schlagkörper aus der Fixierstellung in die Schlagstellung zu überführen, um mit dem Schlagkörper des Schlaginstruments Schlagimpulse auf das distale Ende desselben auszuüben, und zwar wahlweise in distaler oder in proximaler Richtung. Insbesondere kann die Feststelleinrichtung derart ausgebildet sein, dass aktiv eine Betätigung durch einen Anwender erforderlich ist, um die Feststelleinrichtung nicht nur von der Fixierstellung in die Schlagstellung zu überführen, sondern auch in dieser zu halten. Günstig ist es, dass der Schlagkörper in einer Grundstellung relativ zum Schaft festgestellt ist und die mindestens eine Feststellposition einnimmt. So kann ein Anwender das Schlaginstrument insbesondere am Schlagkörper mit einer Hand ergreifen, wobei dann der Schlagkörper relativ zu einem distalen Ende des Schlaginstruments unbeweglich verbleibt. Das Schlaginstrument lässt sich so mit einem medizinischem Objekt auf einfache Weise koppeln. Ferner umfasst die Feststelleinrichtung eine Rückstelleinrichtung zum automatischen Halten der Feststelleinrichtung in der Fixierstellung. Diese Ausgestaltung hat insbesondere den Vorteil, dass der Schlagkörper am Schaft in einer der möglichen Feststellpositionen gehalten ist, ohne dass ein Anwender die Feststelleinrichtung in irgendeiner Art und Weise betätigen muss. So kann insbesondere eine unabsichtliche und unerwünschte Bewegung des Schlagkörpers relativ zum Schaft verhindert werden.

Günstigerweise ist der Schlagkörper in Form eines Handgriffs ausgebildet. Dies ermöglicht es insbesondere, das medizinische Schlaginstrument am Schlagkörper mit einer Hand zu ergreifen und dann mit einer Hand auch handzuhaben, insbesondere um es mit einem medizinischem Objekt zu koppeln. Dafür wird das medizinische Schlaginstrument vorzugsweise mit der Feststelleinrichtung in eine Feststellposition überführt.

Für eine optimale Handhabung ist es vorteilhaft, wenn der Handgriff eine ergonomisch geformte Außenkontur zum Halten desselben mit einer Hand aufweist.

Vorteilhaft ist es, wenn die Feststelleinrichtung ausgebildet ist zum Feststellen des Schlagkörpers am Schaft in der proximalen Anschlagposition und/oder in der distalen Anschlagposition. Die Feststelleinrichtung ist also nicht nur geeignet, in mindestens einer oder beliebig vielen Feststellpositionen zwischen der distalen und der proximalen Anschlagposition den Schlagkörper am Schaft festzustellen, sondern auch in den extremen Stellungen des Schlagkörpers nämlich der proximalen Anschlagposition und der distalen Anschlagposition.

Vorteilhafterweise ist die Feststelleinrichtung entgegen der Wirkung der Rückstelleinrichtung von der Fixierstellung in die Schlagstellung bringbar. Mithin muss also ein Anwender eine Kraft ausüben, um die Feststelleinrichtung insbesondere derart zu betätigen, dass sie von der Fixierstellung in die Schlagstellung überführt wird.

Auf einfache Weise lässt sich die Rückstelleinrichtung ausbilden, wenn sie mindestens ein Rückstellelement umfasst.

Einfach und kostengünstig lässt sich die Rückstelleinrichtung realisieren, wenn das mindestens eine Rückstellelement in Form eines gummi- oder federelastischen Elements ausgebildet ist.

Vorzugsweise ist das mindestens eine Rückstellelement in Form einer Druck- oder Zugfeder ausgebildet. Dies ermöglicht es insbesondere, die Feststelleinrichtung von der Fixierstellung in die Schlagstellung zu überführen durch Ausüben einer Druck- oder Zugkraft entgegen der Wirkung des mindestens einen Rückstellelements.

Gemäß einer weiteren bevorzugten Ausführungsform kann vorgesehen sein, dass die Feststelleinrichtung mindestens ein Feststellelement umfasst zum kraft- und/oder formschlüssigen in Eingriff Bringen sowohl mit dem Schaft als auch mit dem Schlagkörper in der mindestens einen Feststellposition. Diese Ausgestaltung ermöglicht es beispielsweise, das Feststellelement beweglich auszubilden, und zwar sowohl relativ zum Schaft als auch relativ zum Schlagkörper in der Schlagstellung. In der Feststellposition kann dann das mindestens eine Feststellelement insbesondere eine Relativbewegung zwischen dem Schlagkörper und dem Schaft blockieren, beispielsweise durch kraft- und/oder formschlüssigen Eingriff sowohl mit dem Schlagkörper als auch mit dem Schaft.

Auf einfache Weise lässt sich die Rückstelleinrichtung anordnen und ein kompaktes Schlaginstrument ausbilden, wenn sich das mindestens eine Rückstellelement einerseits an einer Feststellelementstützfläche des mindestens einen Feststellelements und andererseits an einer Schlagkörperstützfläche des Schlagkörpers abstützt. Eine solche Ausgestaltung ermöglicht es insbesondere, das mindestens eine Rückstellelement geschützt innerhalb des Schlagkörpers anzuordnen. Innerhalb bedeutet in diesem Sinne innerhalb einer vom Schlagkörper definierten, vorzugsweise tangentenstetigen, Hüllfläche. Ferner wird es so insbesondere auch möglich, das Feststelleelement entgegen der Wirkung des mindestens einen Rückstellelements relativ zum Schlagkörper zu bewegen. Beispielsweise kann der Schlagkörper mit einer Hand ergriffen werden und das mindestens eine Feststelleelement kann durch Betätigen mit einem Finger relativ zum Schlagkörper bewegt, beispielsweise in distaler Richtung verschoben, werden.

Vorteilhaft ist es, wenn die Festelementstützfläche in distaler Richtung weisend ausgebildet ist und wenn die Schlagkörperstützfläche in proximaler Richtung weisend ausgebildet ist. Sowohl die Feststellelementstützfläche als auch die Schlagkörperstützfläche können insbesondere als Ringflächen ausgebildet sein. Dies ermöglicht es insbesondere, die genannten Stützflächen im Zusammenwirken mit einem Rückstellelement in Form einer Schrauben- oder Spiralfeder zu nutzen.

Günstig ist es, wenn das mindestens eine Feststellelement zum Überführen der Feststelleinrichtung von der Fixierstellung in die Schlagstellung relativ zum Schlagkörper bewegbar ist. Insbesondere kann das mindestens eine Feststellelement verschiebbar relativ zum Schlagkörper angeordnet oder ausgebildet sein. Beispielsweise kann das mindestens eine Feststellelement am Schlagkörper bewegbar, insbesondere verschiebbar, gelagert sein. Diese Ausgestaltung ermöglicht insbesondere eine einfache Einhandbedienung durch einen Anwender, welcher den Schlagkörper mit seiner Hand hält und das mindestens eine Feststellelement mit einem Finger, beispielsweise seinem Daumen, verschiebt, um die Feststelleinrichtung von der Fixierstellung in die Schlagstellung zu überführen.

Auf einfache und kompakte Weise lässt sich das Schlaginstrument ausbilden, wenn das mindestens eine Feststelleelement hülsenförmig ausgebildet ist.

Günstig ist es, wenn das mindestens eine Feststellelement zum Überführen der Feststelleinrichtung von der Fixierstellung in die Schlagstellung relativ zum Schlagkörper in distaler Richtung bewegbar ist. Diese Ausgestaltung ermöglicht es insbesondere, dass ein Anwender mit seinem Daumen das mindestens eine Feststellelement in distaler Richtung drückt, während er mit derselben Hand den Schlagkörper hält.

Vorzugsweise steht ein proximales Ende des mindestens einen Feststellelements in der Fixierstellung nicht oder im Wesentlichen nicht über ein proximales Ende des Schlagkörpers vor. Dies ermöglicht es insbesondere, das Schlaginstrument nach Art eines Meisels zu nutzen und beispielsweise mit einem Hammer auf das proximale Ende des Schlagkörpers einzuwirken, ohne dabei das Feststellelement in distaler Richtung zu bewegen, wodurch der relativ zum Schaft festgestellte Schlagkörper in die Schlagstellung überführt würde.

Um insbesondere eine Verletzungsgefahr zu minimieren, beispielsweise auch die Gefahr einer Beschädigung eines Handschuhs eines Anwenders, ist es vorteilhaft, wenn das mindestens eine Feststellelement proximalseitig verschlossen ist. Insbesondere kann das Feststellelement proximalseitig in proximaler Richtung weisend konkav oder konvex gekrümmt geformt sein.

Günstig ist es, wenn der Schlagkörper eine Feststellelementanschlageinrichtung umfasst mit einem distalen und einem proximalen Feststellelementanschlag zum Begrenzen einer Bewegung des mindestens einen Feststellelements in distaler und proximaler Richtung. Eine solche Ausgestaltung ermöglicht eine einfache Handhabung des Schlaginstruments. So kann ein Anwender beispielsweise das mindestens eine Feststellelement zum Überführen des Schlaginstruments von der Fixierstellung in die Schlagstellung einfach gegen den distalen Feststellelementanschlag drücken. Der proximale Feststellelementanschlag begrenzt die Bewegung des mindestens einen Feststellelements in proximaler Richtung. Insbesondere kann die Rückstelleinrichtung das mindestens eine Feststellelement gegen den proximalen Feststellelementanschlag in der Grundstellung drücken.

Vorteilhaft ist es, wenn das mindestens eine Feststellelement eine Feststellelementdurchbrechung umfasst, welche sich quer, insbesondere senkrecht zur Längsachse erstreckt und wenn die Feststellelementanschlageinrichtung die Feststellelementdurchbrechung durchsetzt. Dies ermöglicht insbesondere eine einfache Ausgestaltung des Schlaginstruments. Die Feststellelementdurchbrechung kann beispielsweise in distaler Richtung und in proximaler Richtung weisende Rand- oder Kantenabschnitte umfassen, die im Zusammenwirken mit der Feststellelementanschlageinrichtung die distalen und proximalen Feststellelementanschläge definieren.

Vorzugsweise ist der distale Feststellelementanschlag proximalseitig bezogen auf den proximalen Feststellelementanschlag angeordnet oder ausgebildet.

Dies ermöglicht einen besonders kompakten Aufbau des medizinischen Schlaginstruments.

Günstig ist es, wenn das mindestens eine Feststellelement in Form eines Klemmelements ausgebildet ist und mindestens einen sich in Längsrichtung erstreckenden Klemmarm umfasst und wenn ein freies Ende des mindestens einen Klemmarms in Richtung auf die Längsachse des Schafts hin und von dieser weg bewegbar, insbesondere verschwenkbar, ausgebildet ist. Derartige Feststellelemente ermöglichen es insbesondere auf einfache Weise, eine Klemmung relativ zum Schaft zu reichen. Beispielsweise kann dies bewirkt werden durch Drücken des freien Endes des mindestens einen Klemmarms in Richtung auf die Längsachse des Schafts hin.

Vorzugsweise umfasst der Schlagkörper eine den Schaft umgebende Ausnehmung zum Aufnehmen des mindestens einen Klemmelements. Diese Ausnehmung ermöglicht es insbesondere, dass das freie Ende des mindestens einen Klemmelements vom Schaft weg verschwenkt werden kann, um den Schaft freizugeben. Mit anderen Worten kann so eine Klemmung zwischen dem mindestens einen Feststellelement und dem Schaft aufgehoben werden.

Vorteilhaft ist es, wenn die Ausnehmung mindestens abschnittsweise von einer in Richtung auf die Längsachse hin weisenden Klemmfläche begrenzt ist, welche in der Fixierstellung mit dem mindestens einen Klemmelement zusammenwirkt zum Verhindern einer Bewegung des freien Endes des mindestens einen Klemmelements von der Längsachse weg oder zum klemmenden Halten des mindestens einen Klemmelements in Richtung auf die Längsachse hin. Beispielsweise kann das freie Ende des mindestens einen Klemmelements an der Klemmfläche aufgleiten zum Überführen des Schlaginstruments von der Schlagstellung in die Fixierstellung.

Günstigerweise erweitert sich die Klemmfläche in distaler Richtung konisch und bildet eine Aufgleitfläche. So lässt sich auf einfache Weise eine Relativbewegung zwischen dem freien Ende des mindestens einen Klemmelements und dem Schlagkörper ermöglichen beziehungsweise ausschließen oder blockieren.

Vorteilhaft ist es, wenn am freien Ende des Klemmelements eine von der Längsachse weg weisende, bezogen auf die Längsachse geneigte und mit der Klemmfläche des Schlagkörpers zusammenwirkende Aufgleitfläche ausgebildet ist. Die Aufgleitfläche am freien Ende des Klemmelements kann insbesondere im Zusammenwirken mit der Klemmfläche des Schlagkörpers ein unbeabsichtigtes Verkanten und damit Blockieren der Feststelleinrichtung verhindern helfen.

Vorzugsweise ist ein erster, zwischen der Klemmfläche und der Längsachse definierter Neigungswinkel größer als ein zweiter, zwischen der Aufgleitfläche und der Längsachse definierter Neigungswinkel. Die unterschiedlichen ersten und zweiten Neigungswinkel ermöglichen es insbesondere, eine Selbsthemmung zwischen dem Feststellelement und dem Schlagkörper beim Einsatz des Schlaginstruments zu verhindern. Sie stellen also insbesondere eine zuverlässige Funktionsfähigkeit des Schlaginstruments, insbesondere von dessen Feststelleinrichtung, sicher.

Gemäß einer bevorzugten Ausführungsform kann vorgesehen sein, dass der Schlagkörper eine sich koaxial zur Schaftlängsrichtung erstreckende Längsdurchbrechung aufweist und dass der Schaft mindestens teilweise in der Längsdurchbrechung aufgenommen ist. Beispielsweise kann der proximale Schaftabschnitt vollständig in der Längsdurchbrechung aufgenommen sein, unabhängig von einer Relativposition des Schlagkörpers und des Schafts. Die Längsdurchbrechung kann zudem eine Montage des Schlaginstruments vereinfachen. Optional kann es so für eine Reinigung einfach und schnell zerlegt werden.

Vorteilhaft ist es, wenn der Schaft einen distalen Schaftabschnitt und einen proximalen Schaftabschnitt umfasst, wenn der proximale Schaftabschnitt in der Längsdurchbrechung aufgenommen ist und wenn der distale Schaftabschnitt distalseitig aus der Längsdurchbrechung vorsteht. Eine solche Ausgestaltung ermöglicht es insbesondere, das Schlaginstrument derart zu gestalten, dass der Schlagkörper, unabhängig von einer Relativposition zum Schaft, mit seinem proximalen Ende auch stets das proximale Ende des Schlaginstruments definiert.

Vorteilhaft ist es, wenn eine in Richtung auf die Längsachse hin weisende Innenfläche des mindestens einen Feststellelements eine Führungsfläche für den proximalen Schaftabschnitt bildet. Dies ermöglicht insbesondere, das mindestens eine Feststellelement in definierter Weise relativ zum Schaft zu bewegen, nämlich beispielsweise relativ zum proximalen Schaftabschnitt.

Ferner kann es vorteilhaft sein, wenn die Längsdurchbrechung des Schlagkörpers ausgehend von dessen proximalem Ende eine in Richtung auf die Längsachse hin weisende Führungsfläche für das mindestens eine Feststellelement bildet. Durch diese Führungsfläche kann das mindestens eine Feststellelement in definierter Weise relativ zum Schlagkörper bewegt werden. Insbesondere kann die Führungsfläche derart ausgestaltet sein, dass ein Verkanten des Feststellelements relativ zum Schlagkörper im Bereich der Führungsfläche nicht möglich ist.

Günstig ist es, wenn der proximale Schaftabschnitt den distalen Anschlag umfasst mit einer in proximaler Richtung wirkenden ersten Anschlagfläche und wenn der Schlagkörper eine erste, in distaler Richtung wirkende Schlagkörperanschlagfläche umfasst, welche in der distalen Anschlagposition mit der ersten Anschlagfläche zusammenwirkt. Beispielsweise kann das Schlaginstrument auf einfache Weise realisiert werden, indem eine proximale Endfläche des proximalen Schaftabschnitts die erste Anschlagfläche definiert.

Auf einfache und kompakte Weise lässt sich das Schlaginstrument ausbilden, wenn der Schlagkörper ein Anschlagelement umfasst, welches die erste Schlagkörperanschlagfläche definiert. Das Anschlagelement kann insbesondere nicht nur eine Bewegung des Schafts relativ zum Schlagkörper begrenzen, sondern auch eine Bewegung des mindestens einen Feststellelements relativ zum Schlagkörper.

Vorteilhaft ist es, wenn das Anschlagelement eine Anschlagelementlängsachse definiert und wenn die Anschlagelementlängsachse quer, insbesondere senkrecht, zur Schaftlängsrichtung verläuft. So kann das Anschlagelement beispielsweise quer am Schlagkörper angeordnet oder gehalten sein.

Auf einfache Weise lässt sich das Anschlagelement in Form eines in eine Querbohrung am Schlagkörper eingesetzten Anschlagstiftes ausbilden.

Vorzugsweise ist ein Abstand des Anschlagelements vom proximalen Ende des Schlagkörpers kleiner als ein Abstand vom distalen Ende des Schlagkörpers. Dies ermöglicht einen maximalen Hub des Schafts, insbesondere von dessen proximalem Schaftabschnitt, relativ zum Schlagkörper, insbesondere in der am Schlagkörper ausgebildeten Längsdurchbrechung.

Günstigerweise umfasst die Feststellelementanschlageinrichtung das Anschlagelement. So ist es insbesondere möglich, dass die Feststellelementanschlageinrichtung mehrere Funktionen übernimmt. Auf diese Weise kann ein kompakter Aufbau des Schlaginstruments realisiert werden.

Vorteilhaft ist es, wenn das mindestens eine Rückstellelement distalseitig des Anschlagelements angeordnet oder ausgebildet ist. So kann es beispielsweise in Form eines Druckelements ausgebildet sein, welches durch Bewegen des mindestens einen Feststellelements in distaler Richtung komprimiert wird.

Ferner ist es günstig, wenn der proximale Schaftabschnitt den proximalen Anschlag umfasst mit einer in distaler Richtung wirkenden zweiten Anschlagfläche und wenn der Schlagkörper eine zweite, in proximaler Richtung wirkende Schlagkörperanschlagfläche umfasst, welche in der proximalen Anschlagposition mit der zweiten Anschlagfläche zusammenwirkt. Diese Ausgestaltung ermöglicht insbesondere eine einfache Begrenzung einer Bewegung des Schlagkörpers relativ zum Schaft in proximaler Richtung.

Ein kompakter Aufbau des Schlaginstruments kann insbesondere erreicht werden, wenn die distale Begrenzungswand die zweite Schlagkörperanschlagfläche umfasst.

Vorteilhaft ist es, wenn ein erster Abstand zwischen der ersten Anschlagfläche und der zweiten Anschlagfläche kleiner ist als ein zweiter Abstand zwischen der ersten Schlagkörperanschlagfläche und der zweiten Schlagkörperanschlagfläche. Durch diese Ausgestaltung wird insbesondere eine Bewegung des Schafts relativ zum Schlagkörper ermöglicht. Die Differenz der beiden Abstände bestimmt einen maximalen Hub des Schlagkörpers relativ zum Schaft.

Günstigerweise ist das mindestens eine Rückstellelement das mindestens eine Feststellelement umgebend angeordnet oder ausgebildet ist. So ist es insbesondere möglich, das Rückstellelement geschützt am Schlaginstrument anzuordnen, sodass seine Funktionsfähigkeit dauerhaft gewährleistet werden kann.

Günstigerweise umfasst der Schlagkörper mindestens eine Spülöffnung. Die Spülöffnung kann insbesondere eine Fluidverbindung herstellen zwischen einer Umgebung des Schlagkörpers und der Längsdurchbrechung desselben. So lässt sich das Schlaginstrument auf einfache und sichere Weise reinigen, insbesondere ohne es zu zerlegen.

Vorzugsweise ist die mindestens eine Spülöffnung im Bereich einer die Längsachse umgebenden Umfangswand des Schlagkörpers oder im Bereich einer distalen Begrenzungswand des Schlagkörpers ausgebildet. Mithin ist es also insbesondere möglich, zwei oder mehr Spülöffnungen vorzusehen, und zwar beispielsweise sowohl im Bereich der Umfangswand als auch im Bereich der distalen Begrenzungswand. So kann insbesondere ein Reinigungsfluid das Schlaginstrument zum Reinigen durchströmen, um Verunreinigungen sicher zu entfernen.

Vorteilhaft ist es, wenn die Längsdurchbrechung die distale Begrenzungswand durchsetzt und im Bereich der distalen Begrenzungswand eine Führungsfläche für den distalen Schaftabschnitt bildet. Auf diese Weise kann insbesondere eine Bewegung des Schlagkörpers relativ zum Schaft einfach und definiert geführt werden. Die Führungsfläche kann insbesondere umlaufend ausgebildet sein. Sie kann auch durch mindestens eine Spülöffnung unterbrochen sein. Die Führungsfläche kann insbesondere hohlzylindrisch ausgebildet sein oder hohlzylindrische Flächenabschnitte umfassen.

Gemäß einer weiteren bevorzugten Ausführungsform kann vorgesehen sein, dass das Schlaginstrument eine Kopplungseinrichtung umfasst zum temporären Koppeln mit einem medizinischen Objekt. Wie eingangs erläutert kann so das Schlaginstrument beispielsweise mit einem Befestigungselement gekoppelt werden, um es aus einem Knochen, in dem es verankert wurde, auszuschlagen. Hierzu werden Schlagimpulse vom Schlagkörper auf den Schaft in der Schlagstellung übertragen, und zwar dann, wenn der Schlagkörper mit dem proximalen Anschlag, welcher die proximale Anschlagposition definiert, zusammenwirkt.

Vorteilhaft ist es, wenn das medizinische Objekt in Form eines medizinischen Implantats ausgebildet ist. Beispielsweise kann es in Form eine medizinischen Befestigungselements oder in Form einer Implantatkomponente eines Gelenkimplantats ausgebildet sein. Das medizinische Schlaginstrument kann so insbesondere zum Verankern oder auch wieder zum Lösen der medizinischen Objekte an beziehungsweise aus einem Knochen eingesetzt werden. Ferner kann so auch ein medizinisches System umfassend ein medizinisches Schlaginstrument und mindestens ein medizinisches Objekt definiert werden.

Günstig ist es, wenn die Kopplungseinrichtung ein erstes Kopplungselement umfasst, welches ausgebildet ist zum kraft- und/oder formschlüssigen in Eingriff Bringen mit einem korrespondierenden zweiten, vom medizinischen Objekt umfassten Kopplungselement in einer Kopplungsstellung. Die Kopplungseinrichtung ermöglicht insbesondere eine einfache und sichere Verbindung des Schlaginstruments mit einem medizinischen Objekt in der Kopplungsstellung.

Die Kopplungseinrichtung lässt sich auf einfache Weise ausbilden, wenn das erste Kopplungselement in Form eines Kopplungsvorsprungs oder einer Kopplungsaufnahme ausgebildet ist. Das zweite Kopplungselement kann dann korrespondierend zum ersten Kopplungselement in Form einer Kopplungsaufnahme beziehungsweise eines Kopplungsvorsprungs ausgebildet sein.

Günstig ist es, wenn die Kopplungseinrichtung eine Sicherungseinrichtung umfasst zum kraft- und/oder formschlüssigen Sichern eines medizinischen Objekts am medizinischen Schlaginstrument in der Kopplungsstellung. Das Schlaginstrument kann mittels der Kopplungseinrichtung mit dem medizinischen Instrument gekoppelt werden. Unabhängig davon dient die Sicherungseinrichtung dazu, das medizinische Objekt am Schlaginstrument in der Kopplungsstellung zu sichern, beispielsweise gegen ein unbeabsichtigtes Lösen. So kann insbesondere eine zuverlässige Handhabung des Schlaginstruments erreicht werden. So kann insbesondere auch ein Risiko deutlich verringert werden, dass das mit dem Schlaginstrument gekoppelte medizinische Objekt in unerwünschter Weise vom Schlaginstrument abfallen kann.

Günstig ist es, wenn die Sicherungseinrichtung von einer Freigabestellung, in welcher das medizinische Schlaginstrument und das medizinische Objekt in einer Eingriffsrichtung in Eingriff bringbar sind, in eine Sicherungsstellung bringbar ist, in welcher eine Bewegung des medizinischen Schlaginstruments und des medizinischen Objekts relativ zueinander in oder entgegengesetzt zur Eingriffsrichtung blockiert ist. Mit anderen Worten wirken die Sicherungseinrichtung und die Kopplungseinrichtung in linear unabhängigen Richtungen, sodass insbesondere ein unbeabsichtigtes Lösen des medizinischen Objekts vom Schlaginstrument nur nach Überwinden einer Freigabekraft zum Deaktivieren der Sicherungseinrichtung möglich ist.

Auf einfache Weise lässt sich das Schlaginstrument realisieren, wenn die Eingriffsrichtung quer, insbesondere senkrecht, zur Schaftlängsrichtung verläuft. Wie bereits angedeutet können so linear unabhängige Bewegungen realisiert werden, um das medizinische Objekt in definierter Weise am Schaft in der Kopplungsstellung zu sichern.

Vorteilhaft ist es, wenn die Sicherungseinrichtung ein Sicherungsglied umfasst, welches mit einem Sicherungselement des medizinischen Objekts in der Sicherungsstellung kraft- und/oder formschlüssig in Eingriff steht. Das Sicherungsglied kann also das medizinische Objekt insbesondere in definierter Weise in der Kopplungsstellung am Schaft sichern, und zwar wenn das Sicherungsglied nicht die Freigabestellung, sondern die Sicherungsstellung einnimmt.

Günstigerweise ist das Sicherungsglied am Schaft bewegbar, insbesondere verschiebbar, gelagert. So lässt sich ein einfacher Sicherungsmechanismus für das medizinische Objekt am Schlaginstrument realisieren.

Vorteilhaft ist es, wenn die Sicherungseinrichtung einen Sicherungsgliedanschlag umfasst, an welchem das Sicherungsglied in der Sicherungsstellung anschlägt. Der Sicherungsgliedanschlag kann insbesondere dem Zweck dienen, dass das Sicherungsglied nicht in unerwünschter Weise in distaler Richtung vom Schaft abfallen kann. Mithin dient der Sicherungsgliedanschlag also einer Begrenzung einer Bewegung des Sicherungsglieds, insbesondere in distaler Richtung.

Die Sicherungseinrichtung lässt sich auf einfache Weise realisieren, wenn der Sicherungsgliedanschlag eine in proximaler Richtung weisende Anschlagfläche umfasst. So kann eine Bewegung des Sicherungsglieds in distaler Richtung blockiert werden, wenn es mit der Anschlagfläche des Sicherungsgliedanschlags zusammenwirkt.

Günstig ist es, wenn das Sicherungsglied einen Sicherungsgliedschaft und einen proximalseitig an diesem angeordneten oder ausgebildeten Sicherungsgliedkopf umfasst und wenn der Sicherungsgliedkopf mit dem Sicherungsgliedanschlag zusammenwirkend ausgebildet ist. Mit anderen Worten bildet der Sicherungsgliedkopf ebenfalls einen Anschlag oder eine Anschlagfläche, die mit dem Sicherungsgliedanschlag am Schaft zusammenwirkt, um insbesondere eine Bewegung des Sicherungsglieds in distaler Richtung zu begrenzen.

Vorteilhaft ist es, wenn der Sicherungsgliedkopf eine in distaler Richtung weisende, mit der Anschlagfläche des Sicherungsgliedanschlags zusammenwirkende Sicherungsgliedkopfanschlagfläche umfasst. So kann insbesondere auf einfache Weise eine Bewegung des Sicherungsglieds in distaler Richtung begrenzt werden.

Auf einfache Weise lässt sich die Bewegung des Sicherungsglieds in distaler Richtung begrenzen, wenn die Anschlagfläche in Form einer Ringfläche ausgebildet ist. Die Sicherungsgliedkopfanschlagfläche kann ebenfalls in Form einer Ringfläche ausgebildet sein.

Ferner kann vorgesehen sein, dass das Sicherungsglied ein distales, mit dem medizinischen Objekt in Eingriff bringbares Sicherungsgliedende umfasst und dass das distale Sicherungsgliedende in distaler Richtung weisend abgerundet ist. So kann insbesondere eine Verletzungsgefahr beim Einsatz des Schlaginstruments vermieden werden. Zudem kann das abgerundete Sicherungsgliedende ein Verkanten mit einem Vorsprung oder einer Ausnehmung am medizinischen Objekt verhindern, beispielsweise beim Koppeln oder Entkoppeln derselben.

Günstigerweise umfasst die Sicherungseinrichtung eine Vorspanneinrichtung zum automatischen Überführen der Sicherungseinrichtung von der Freigabestellung in die Sicherungsstellung. Durch die Vorspanneinrichtung wird also insbesondere erreicht, dass die Sicherungseinrichtung ohne zusätzliche, von außen einwirkenden Kräften stets die Sicherungsstellung einnimmt. Beispielsweise kann die Sicherungseinrichtung mittels der Vorspanneinrichtung in Form einer Rast- oder Schnappverbindungseinrichtung ausgebildet werden, die ein einfaches, insbesondere werkzeugfreies Koppeln des Schlaginstruments mit dem medizinischen Objekt ermöglicht.

Vorteilhaft ist es, wenn die Vorspanneinrichtung ausgebildet ist zum Halten des Sicherungsglieds in der Sicherungsstellung unter Vorspannung. So lässt sich das medizinische Objekt insbesondere einfach und sicher mit der Sicherungseinrichtung am Schlaginstrument in der Kopplungsstellung sichern.

Auf einfache Weise lässt sich die Vorspanneinrichtung ausbilden, wenn sie mindestens ein vorspannendes Element umfasst.

Günstigerweise ist das mindestens eine vorspannende Element in Form eines gummi- oder federelastischen Elements ausgebildet. Diese lassen sich einfach und kostengünstig herstellen, beispielweise auch aus heißdampfsterilisierbaren Materialien.

Je nach Ausgestaltung der Sicherungseinrichtung kann es vorteilhaft sein, wenn das vorspannende Element in Form einer Druck- oder Zugfeder ausgebildet ist.

Vorzugsweise stützt sich das mindestens eine vorspannende Element einerseits am Sicherungsglied und andererseits an einer in distaler Richtung wirkenden Stützfläche ab. Eine solche Ausgestaltung ist insbesondere beim Einsatz eines vorspannenden Elements in Form eines Druckelements, also beispielsweise einer Druckfeder, vorteilhaft.

Günstig ist es, wenn die Sicherungseinrichtung ein am Schaft angeordnetes Stützelement umfasst und wenn das Stützelement die Stützfläche umfasst. Das Stützelement mit der Stützfläche bildet so insbesondere einen Anschlag für das vorspannende Element und damit insbesondere auch einen Anschlag für eine Bewegung des Sicherungsglied in proximaler Richtung.

Auf einfache Weise lässt sich das Stützelement ausbilden, wenn es eine Stützelementlängsachse definiert und wenn die Stützelementlängsachse quer, insbesondere senkrecht, zur Schaftlängsrichtung verläuft.

Einfach und kostengünstig lässt sich das Schlaginstrument ausbilden, wenn das Stützelement in Form eines in eine Querbohrung am Schaft eingesetzten Stiftes ausgebildet ist. Der Stift kann beispielsweise in die Querbohrung eingeschraubt oder/oder stoffschlüssig mit dem Schaft verbunden sein beispielsweise durch Kleben, Löten oder Schweißen.

Die vorstehende Beschreibung umfasst somit insbesondere die nachfolgend in Form durchnummerierter Sätze definierten Ausführungsformen medizinischer Schlaginstrumente, wobei die durchnummerierten Sätze nicht den Schutzbereich der mit den Patentansprüchen beanspruchten Erfindung definieren:
1. Medizinisches Schlaginstrument (12), insbesondere in Form eines Schlaghammers (16), mit einem eine Schaftlängsrichtung (20) sowie ein proximales und ein distales Ende (22, 24) definierenden Schaft (18) und einem am Schaft (18) angeordneten, eine Längsachse (28) definierenden Schlagkörper (26), wobei der Schlagkörper (26) in einer Schlagstellung am Schaft (18) zwischen einem eine distale Anschlagposition des Schlagkörpers (26) definierenden distalen Anschlag (30) und einem eine proximale Anschlagposition des Schlagkörpers (26) definierenden proximalen Anschlag (32) bewegbar, insbesondere verschiebbar, ist zum Übertragen eines Schlagimpulses auf das distale Ende (24) des Schafts (18) in distaler beziehungsweise proximaler Richtung, dadurch gekennzeichnet, dass das Schlaginstrument (12) eine Feststelleinrichtung (34) umfasst zum temporären Feststellen des Schlagkörpers (26) am Schaft (18) in mindestens einer, insbesondere beliebigen, Feststellposition zwischen der distalen Anschlagposition und der proximalen Anschlagposition.
2. Medizinisches Schlaginstrument nach Satz 1, dadurch gekennzeichnet, dass der Schlagkörper (26) in Form eines Handgriffs (126) ausgebildet ist.
3. Medizinisches Schlaginstrument nach Satz 2, dadurch gekennzeichnet, dass der Handgriff (126) eine ergonomisch geformte Außenkontur (128) zum Halten desselben mit einer Hand (130) aufweist.
4. Medizinisches Schlaginstrument nach einem der voranstehenden Sätze, dadurch gekennzeichnet, dass der Schlagkörper (26) in einer Grundstellung relativ zum Schaft (18) festgestellt ist und die mindestens eine Feststellposition einnimmt.
5. Medizinisches Schlaginstrument nach einem der voranstehenden Sätze, dadurch gekennzeichnet, dass die Feststelleinrichtung (34) ausgebildet ist zum Feststellen des Schlagkörpers (26) am Schaft (18) in der proximalen Anschlagposition und/oder in der distalen Anschlagposition.
6. Medizinisches Schlaginstrument nach einem der voranstehenden Sätze, dadurch gekennzeichnet, dass die Feststelleinrichtung (34) von einer Fixierstellung, in welcher der Schlagkörper (26) in einer der beliebigen Feststellpositionen am Schaft (18) festgelegt ist, in die Schlagstellung, in welcher der Schlagkörper (26) und der Schaft (18) relativ zueinander bewegbar sind, überführbar ist und umgekehrt.
7. Medizinisches Schlaginstrument nach Satz 6, dadurch gekennzeichnet, dass die Feststelleinrichtung (34) eine Rückstelleinrichtung (214) umfasst zum automatischen Halten der Feststelleinrichtung (34) in der Fixierstellung.
8. Medizinisches Schlaginstrument nach Satz 7, dadurch gekennzeichnet, dass die Feststelleinrichtung (34) entgegen der Wirkung der Rückstelleinrichtung (214) von der Fixierstellung in die Schlagstellung bringbar ist.
9. Medizinisches Schlaginstrument nach Satz 7 oder 8, dadurch gekennzeichnet, dass die Rückstelleinrichtung (214) mindestens ein Rückstellelement (216) umfasst.
10. Medizinisches Schlaginstrument nach Satz 9, dadurch gekennzeichnet, dass das mindestens eine Rückstellelement (216) in Form eines gummi- oder federelastischen Elements (218) ausgebildet ist.
11. Medizinisches Schlaginstrument nach Satz 9 oder 10, dadurch gekennzeichnet, dass mindestens eine Rückstellelement (216) in Form einer Druck- oder Zugfeder (220) ausgebildet ist.
12. Medizinisches Schlaginstrument nach einem der voranstehenden Sätze, dadurch gekennzeichnet, dass die Feststelleinrichtung (34) mindestens ein Feststellelement (172) umfasst zum kraft- und/oder formschlüssigen in Eingriff Bringen sowohl mit dem Schaft (18) als auch mit dem Schlagkörper (26) in der mindestens einen Feststellposition.
13. Medizinisches Schlaginstrument nach Satz 12, dadurch gekennzeichnet, dass sich das mindestens eine Rückstellelement (216) einerseits an einer Feststellelementstützfläche (188) des mindestens einen Feststellelements (172) und andererseits an einer Schlagkörperstützfläche (156) des Schlagkörpers (26) abstützt.
14. Medizinisches Schlaginstrument nach Satz 13, dadurch gekennzeichnet, dass die Feststellelementstützfläche (188) in distaler Richtung weisend ausgebildet ist, insbesondere als Ringfläche, und dass die Schlagkörperstützfläche (156) in proximaler Richtung weisend ausgebildet ist, insbesondere als Ringfläche (158).
15. Medizinisches Schlaginstrument nach einem der Sätze 12 bis 14 dadurch gekennzeichnet, dass das mindestens eine Feststellelement (172) zum Überführen der Feststelleinrichtung (34) von der Fixierstellung in die Schlagstellung relativ zum Schlagkörper (26) bewegbar, insbesondere verschiebbar, ist.
16. Medizinisches Schlaginstrument nach einem der Sätze 12 bis 15, dadurch gekennzeichnet, dass das mindestens eine Feststellelement (172) hülsenförmig ausgebildet ist.
17. Medizinisches Schlaginstrument nach einem der Sätze 12 bis 16, dadurch gekennzeichnet, dass das mindestens eine Feststellelement (172) zum Überführen der Feststelleinrichtung (34) von der Fixierstellung in die Schlagstellung relativ zum Schlagkörper (26) in distaler Richtung bewegbar ist.
18. Medizinisches Schlaginstrument nach einem der Sätze 12 bis 17, dadurch gekennzeichnet, dass ein proximales Ende (176) des mindestens einen Feststellelements (172) in der Fixierstellung nicht oder im Wesentlichen nicht über ein proximales Ende (168) des Schlagkörpers (26) vorsteht.
19. Medizinisches Schlaginstrument nach einem der Sätze 12 bis 18, dadurch gekennzeichnet, dass das mindestens eine Feststellelement (172) proximalseitig verschlossen ist.
20. Medizinisches Schlaginstrument nach einem der Sätze 12 bis 19, dadurch gekennzeichnet, dass der Schlagkörper (26) eine Feststellelementanschlageinrichtung (204) umfasst mit einem distalen und einem proximalen Feststellelementanschlag (206, 208) zum Begrenzen einer Bewegung des mindestens einen Feststellelements (172) in distaler und proximaler Richtung.
21. Medizinisches Schlaginstrument nach Satz 20, dadurch gekennzeichnet, dass das mindestens eine Feststellelement (172) eine Feststellelementdurchbrechung (202) umfasst, welche sich quer, insbesondere senkrecht, zur Längsachse (28) erstreckt, und dass die Feststellelementanschlageinrichtung (204) die Feststellelementdurchbrechung (202) durchsetzt.
22. Medizinisches Schlaginstrument nach Satz 20 oder 21, dadurch gekennzeichnet, dass der distale Feststellelementanschlag (206) proximalseitig bezogen auf den proximalen Feststellelementanschlag (208) angeordnet oder ausgebildet ist.
23. Medizinisches Schlaginstrument nach einem der Sätze 12 bis 22, dadurch gekennzeichnet, dass das mindestens eine Feststellelement (172) in Form eines Klemmelements (196) ausgebildet ist und mindestens einen sich in Längsrichtung erstreckenden Klemmarm (200) umfasst und dass ein freies Ende (222) des mindestens einen Klemmarms (200) in Richtung auf die Längsachse (28) des Schafts (26) hin und von dieser weg bewegbar, insbesondere verschwenkbar, ausgebildet ist.
24. Medizinisches Schlaginstrument nach Satz 23, dadurch gekennzeichnet, dass der Schlagkörper (26) eine den Schaft (18) umgebende Ausnehmung (150) zum Aufnehmen des mindestens eine Klemmelements (196) umfasst.
25. Medizinisches Schlaginstrument nach Satz 24, dadurch gekennzeichnet, dass die Ausnehmung (150) mindestens abschnittsweise von einer in Richtung auf die Längsachse (28) hin weisenden Klemmfläche (152) begrenzt ist, welche in der Fixierstellung mit dem mindestens einen Klemmelement (196) zusammenwirkt zum Verhindern einer Bewegung des freien Endes (222) des mindestens einen Klemmelements (196) von der Längsachse (28) weg oder zum klemmenden Halten des mindestens einen Klemmelements (196) in Richtung auf die Längsachse (28) hin.
26. Medizinisches Schlaginstrument nach Satz 25, dadurch gekennzeichnet, dass sich die Klemmfläche (152) in distaler Richtung konisch erweitert und eine Aufgleitfläche bildet.
27. Medizinisches Schlaginstrument nach Satz 25 oder 26, dadurch gekennzeichnet, dass am freien Ende (222) des Klemmelements (196) eine von der Längsachse (28) weg weisende, bezogen auf die Längsachse (28) geneigte und mit der Klemmfläche (152) des Schlagkörpers (26) zusammenwirkende Aufgleitfläche (192) ausgebildet ist.
28. Medizinisches Schlaginstrument nach Satz 27, dadurch gekennzeichnet, dass ein erster, zwischen der Klemmfläche (152) und der Längsachse (28) definierter Neigungswinkel (224) größer ist als ein zweiter, zwischen der Aufgleitfläche (192) und der Längsachse (28) definierter Neigungswinkel (226).
29. Medizinisches Schlaginstrument nach einem der voranstehenden Sätze, dadurch gekennzeichnet, dass der Schlagkörper (26) eine sich koaxial zur Schaftlängsrichtung (20) erstreckende Längsdurchbrechung (138) aufweist und dass der Schaft (18) mindestens teilweise in der Längsdurchbrechung (138) aufgenommen ist.
30. Medizinisches Schlaginstrument nach Satz 29, dadurch gekennzeichnet, dass der Schaft (18) einen distalen Schaftabschnitt (36) und einen proximalen Schaftabschnitt (38) umfasst, dass der proximale Schaftabschnitt (38) in der Längsdurchbrechung (138) aufgenommen ist und dass der distale Schaftabschnitt (36) distalseitig aus der Längsdurchbrechung (138) vorsteht.
31. Medizinisches Schlaginstrument nach Satz 29 oder 30, dadurch gekennzeichnet, dass eine in Richtung auf die Längsachse (28) hin weisende Innenfläche (228) des mindestens einen Feststellelements (172) eine Führungsfläche (230) für den proximalen Schaftabschnitt (38) bildet.
32. Medizinisches Schlaginstrument nach einem der Sätze 29 bis 31, dadurch gekennzeichnet, dass die Längsdurchbrechung (138) des Schlagkörpers (26) ausgehend von dessen proximalem Ende eine in Richtung auf die Längsachse (28) hin weisende Führungsfläche (232) für das mindestens eine Feststellelement (172) bildet.
33. Medizinisches Schlaginstrument nach Satz 32, dadurch gekennzeichnet, dass der proximale Schaftabschnitt (22) den distalen Anschlag (30) umfasst mit einer in proximaler Richtung wirkenden ersten Anschlagfläche (44) und dass der Schlagkörper (26) eine erste, in distaler Richtung wirkende Schlagkörperanschlagfläche (234) umfasst, welche in der distalen Anschlagposition mit der ersten Anschlagfläche (44) zusammenwirkt.
34. Medizinisches Schlaginstrument nach Satz 33, dadurch gekennzeichnet, dass der Schlagkörper (36) ein Anschlagelement (160) umfasst, welches die erste Schlagkörperanschlagfläche (234) definiert.
35. Medizinisches Schlaginstrument nach Satz 34, dadurch gekennzeichnet, dass das Anschlagelement (160) eine Anschlagelementlängsachse (162) definiert und dass die Anschlagelementlängsachse (162) quer, insbesondere senkrecht, zur Schaftlängsrichtung (20) verläuft.
36. Medizinisches Schlaginstrument nach ein Satz 34 oder 35, dadurch gekennzeichnet, dass das Anschlagelement (160) in Form eines in eine Querbohrung (164) am Schlagkörper (26) eingesetzten Anschlagstiftes (166) ausgebildet ist.
37. Medizinisches Schlaginstrument nach einem der Sätze 34 bis 36, dadurch gekennzeichnet, dass ein Abstand des Anschlagelements (160) vom proximalen Ende (168) des Schlagkörpers (26) kleiner ist als ein Abstand vom distalen Ende (170) des Schlagkörpers (26).
38. Medizinisches Schlaginstrument nach einem der Sätze 34 bis 37, dadurch gekennzeichnet, dass die Feststellelementanschlageinrichtung (204) das Anschlagelement (160) umfasst.
39. Medizinisches Schlaginstrument nach einem der Sätze 34 bis 38, dadurch gekennzeichnet, dass das mindestens eine Rückstellelement (216) distalseitig des Anschlagelements (160) angeordnet oder ausgebildet ist.
40. Medizinisches Schlaginstrument nach einem der Sätze 31 bis 39, dadurch gekennzeichnet, dass der proximale Schaftabschnitt (38) den proximalen Anschlag (32) umfasst mit einer in distaler Richtung wirkenden zweiten Anschlagfläche (48) und dass der Schlagkörper (26) eine zweite, in proximaler Richtung wirkende Schlagkörperanschlagfläche (236) umfasst, welche in der proximalen Anschlagposition mit der zweiten Anschlagfläche (48) zusammenwirkt.
41. Medizinisches Schlaginstrument nach Satz 40, dadurch gekennzeichnet, dass die distale Begrenzungswand (146) die zweite Schlagkörperanschlagfläche (236) umfasst.
42. Medizinisches Schlaginstrument nach Satz 40 oder 41, dadurch gekennzeichnet, dass ein erster Abstand (240) zwischen der ersten Anschlagfläche (44) und der zweiten Anschlagfläche (48) kleiner ist als ein zweiter Abstand (242) zwischen der ersten Schlagkörperanschlagfläche (234) und der zweiten Schlagkörperanschlagfläche (236).
43. Medizinisches Schlaginstrument nach einem der Sätze 12 bis 42, dadurch gekennzeichnet, dass das mindestens eine Rückstellelement (216) das mindestens eine Feststellelement (172) umgebend angeordnet oder ausgebildet ist.
44. Medizinisches Schlaginstrument nach einem der voranstehenden Sätze, dadurch gekennzeichnet, dass der Schlagkörper (26) mindestens eine Spülöffnung (244, 246) umfasst.
45. Medizinisches Schlaginstrument nach Satz 44, dadurch gekennzeichnet, dass die mindestens eine Spülöffnung (244, 246) im Bereich einer die Längsachse (28) umgebenden Umfangswand (144) des Schlagkörpers (26) oder im Bereich einer distalen Begrenzungswand (146) des Schlagkörpers (26) ausgebildet ist.
46. Medizinisches Schlaginstrument nach Satz 45, dadurch gekennzeichnet, dass die Längsdurchbrechung (138) die distale Begrenzungswand (146) durchsetzt und im Bereich der distalen Begrenzungswand (146) eine Führungsfläche (148) für den distalen Schaftabschnitt (36) bildet.
47. Medizinisches Schlaginstrument nach einem der voranstehenden Sätze, dadurch gekennzeichnet, dass das Schlaginstrument (12) eine Kopplungseinrichtung (58) umfasst zum temporären Koppeln mit einem medizinischen Objekt (14).
48. Medizinisches Schlaginstrument nach Satz 47, dadurch gekennzeichnet, dass das medizinische Objekt (14) in Form eines medizinischen Implantats (60) ausgebildet ist, insbesondere in Form eines medizinischen Befestigungselements (62) oder in Form einer Implantatkomponente eines Gelenkimplantats.
49. Medizinisches Schlaginstrument nach Satz 48 oder 49, dadurch gekennzeichnet, dass die Kopplungseinrichtung (58) ein erstes Kopplungselement (64) umfasst, welches ausgebildet ist zum kraft- und/oder formschlüssigen in Eingriff Bringen mit einem korrespondierenden zweiten, vom medizinischen Objekt (14) umfassten Kopplungselement (66) in einer Kopplungsstellung.
50. Medizinisches Schlaginstrument nach Satz 49, dadurch gekennzeichnet, dass das erste Kopplungselement (64) in Form eines Kopplungsvorsprungs oder einer Kopplungsaufnahme (68) ausgebildet ist.
51. Medizinisches Schlaginstrument nach einem der Sätze 47 bis 50, dadurch gekennzeichnet, dass die Kopplungseinrichtung (58) eine Sicherungseinrichtung (94) umfasst zum kraft- und/oder formschlüssigen Sichern eines medizinischen Objekts (14) am medizinischen Schlaginstrument (12) in der Kopplungsstellung.
52. Medizinisches Schlaginstrument nach Satz 51, dadurch gekennzeichnet, dass die Sicherungseinrichtung (94) von einer Freigabestellung, in welcher das medizinische Schlaginstrument (12) und das medizinische Objekt (14) in einer Eingriffsrichtung in Eingriff bringbar sind, in eine Sicherungsstellung (90) bringbar ist, in welcher eine Bewegung des medizinischen Schlaginstruments (12) und des medizinischen Objekts (14) relativ zueinander in oder entgegengesetzt zur Eingriffsrichtung (90) blockiert ist.
53. Medizinisches Schlaginstrument nach Satz 52, dadurch gekennzeichnet, dass die Eingriffsrichtung (90) quer, insbesondere senkrecht, zur Schaftlängsrichtung (20) verläuft.
54. Medizinisches Schlaginstrument nach Satz 52 oder 53, dadurch gekennzeichnet, dass die Sicherungseinrichtung (94) ein Sicherungsglied (96) umfasst, welches mit einem Sicherungselement (98) des medizinischen Objekts (14) in der Sicherungsstellung kraft- und/oder formschlüssig in Eingriff steht.
55. Medizinisches Schlaginstrument nach Satz 54, dadurch gekennzeichnet, dass das Sicherungsglied (96) am Schaft (18) bewegbar, insbesondere verschiebbar, gelagert ist.
56. Medizinisches Schlaginstrument nach Satz 54 oder 55, dadurch gekennzeichnet, dass die Sicherungseinrichtung (94) einen Sicherungsgliedanschlag (110) umfasst, an welchem das Sicherungsglied (96) in der Sicherungsstellung anschlägt.
57. Medizinisches Schlaginstrument nach Satz 56, dadurch gekennzeichnet, dass der Sicherungsgliedanschlag (110) eine in proximaler Richtung weisende Anschlagfläche (112) umfasst.
58. Medizinisches Schlaginstrument nach Satz 56 oder 57, dadurch gekennzeichnet, dass das Sicherungsglied (94) einen Sicherungsgliedschaft (102) und einen proximalseitig an diesem angeordneten oder ausgebildeten Sicherungsgliedkopf (104) umfasst und dass der Sicherungsgliedkopf (104) mit dem Sicherungsgliedanschlag (110) zusammenwirkend ausgebildet ist.
59. Medizinisches Schlaginstrument nach Satz 58, dadurch gekennzeichnet, dass der Sicherungsgliedkopf (104) eine in distaler Richtung weisende, mit der Anschlagfläche (112) des Sicherungsgliedanschlags (110) zusammenwirkende Sicherungsgliedkopfanschlagfläche (106) umfasst.
60. Medizinisches Schlaginstrument nach einem der Sätze 57 bis 59, dadurch gekennzeichnet, dass die Anschlagfläche (112) in Form einer Ringfläche (114) ausgebildet ist.
61. Medizinisches Schlaginstrument nach einem der Sätze 54 bis 60, dadurch gekennzeichnet, dass das Sicherungsglied (96) ein distales, mit dem medizinischen Objekt (14) in Eingriff bringbares Sicherungsgliedende (108) umfasst und dass das distale Sicherungsgliedende (108) in distaler Richtung weisend abgerundet ist.
62. Medizinisches Schlaginstrument nach einem der Sätze 51 bis 61, dadurch gekennzeichnet, dass die Sicherungseinrichtung (94) eine Vorspanneinrichtung (116) umfasst zum automatischen Überführen der Sicherungseinrichtung (94) von der Freigabestellung in die Sicherungsstellung.
63. Medizinisches Schlaginstrument nach Satz 62, dadurch gekennzeichnet, dass die Vorspanneinrichtung (116) ausgebildet ist zum Halten des Sicherungsglieds (96) in der Sicherungsstellung unter Vorspannung.
64. Medizinisches Schlaginstrument nach Satz 62 oder 63, dadurch gekennzeichnet, dass die Vorspanneinrichtung (116) mindestens ein vorspannendes Element (118) umfasst.
65. Medizinisches Schlaginstrument nach Satz 64, dadurch gekennzeichnet, dass das mindestens eine vorspannende Element (118) in Form eines gummi- oder federelastischen Elements (120) ausgebildet ist.
66. Medizinisches Schlaginstrument nach Satz 64 oder 65, dadurch gekennzeichnet, dass vorspannende Element (118) in Form einer Druck- oder Zugfeder (122) ausgebildet ist.
67. Medizinisches Schlaginstrument nach einem der Sätze 64 bis 66, dadurch gekennzeichnet, dass sich das mindestens eine vorspannende Element (118) einerseits am Sicherungsglied (96) und andererseits an einer in distaler Richtung wirkenden Stützfläche (124) abstützt.
68. Medizinisches Schlaginstrument nach Satz 67, dadurch gekennzeichnet, dass die Sicherungseinrichtung (94) ein am Schaft (18) angeordnetes Stützelement (52) umfasst und dass das Stützelement (52) die Stützfläche (124) umfasst.
69. Medizinisches Schlaginstrument nach Satz 68, dadurch gekennzeichnet, dass das Stützelement (52) eine Stützelementlängsachse (56) definiert und dass die Stützelementlängsachse (56) quer, insbesondere senkrecht, zur Schaftlängsrichtung (20) verläuft.
70. Medizinisches Schlaginstrument nach Satz 68 oder 69, dadurch gekennzeichnet, dass das Stützelement (52) in Form eines in eine Querbohrung (50) am Schaft (18) eingesetzten Stiftes (54) ausgebildet ist.

Die nachfolgende Beschreibung bevorzugter Ausführungsformen der Erfindung dient im Zusammenhang mit den Zeichnungen der näheren Erläuterung. Es zeigen:
- Figur 1:: eine schematische, teilweise durchbrochene Gesamtansicht eines Ausführungsbeispiels eines medizinischen Systems umfassend ein medizinisches Schlaginstrument mit daran gekoppeltem medizinischem Objekt;
- Figur 2:: eine perspektivische, teilweise durchbrochene Ansicht eines distalen Endes des Schlaginstruments aus Figur 1 mit von diesem getrenntem medizinischen Objekt;

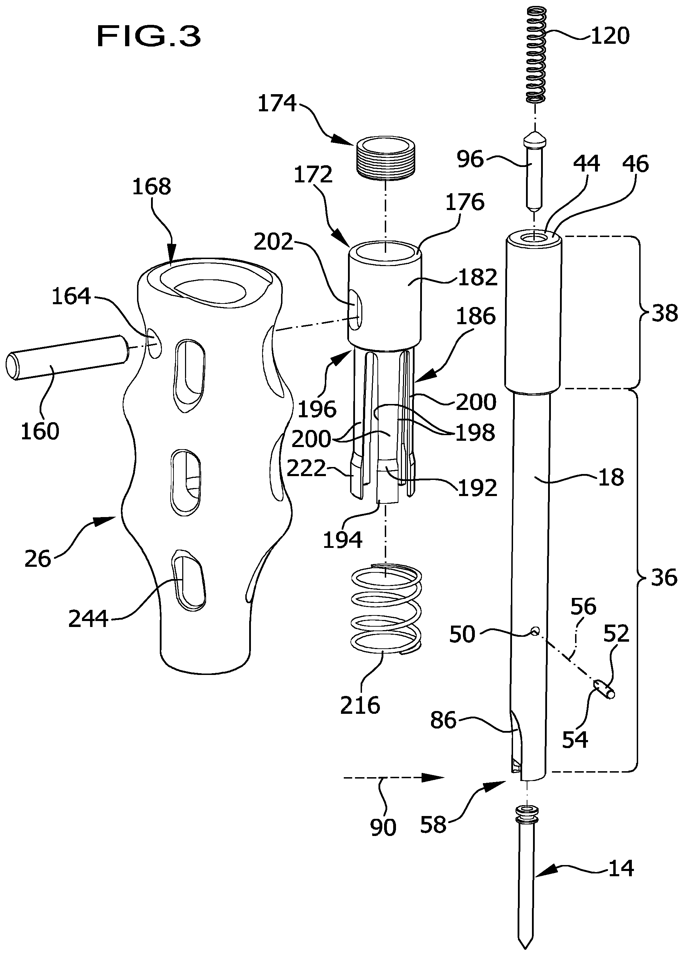

- Figur 3:: eine perspektivische Explosionsdarstellung der Anordnung aus Figur 1;

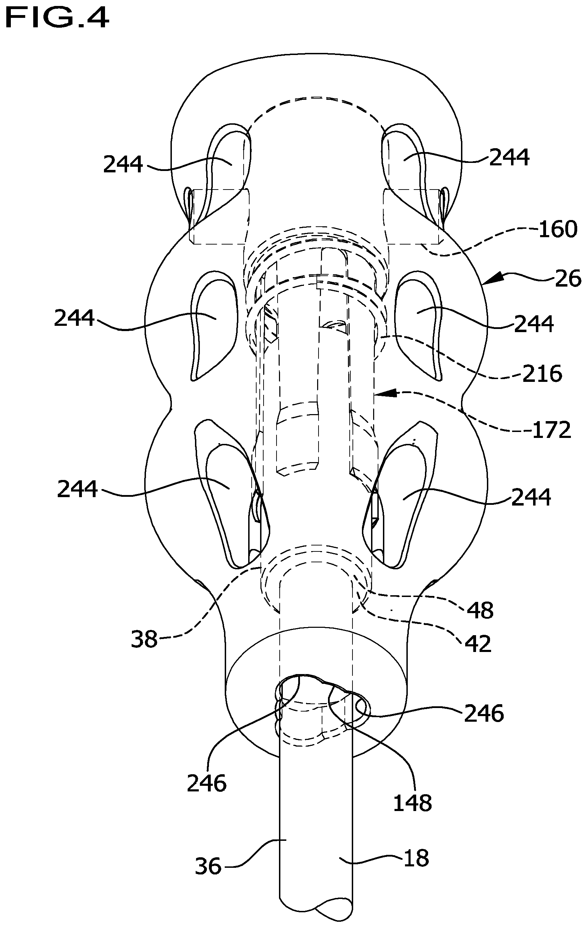

- Figur 4:: eine perspektivische Ansicht eines proximalen Endbereichs des Schlaginstruments aus Figur 1;

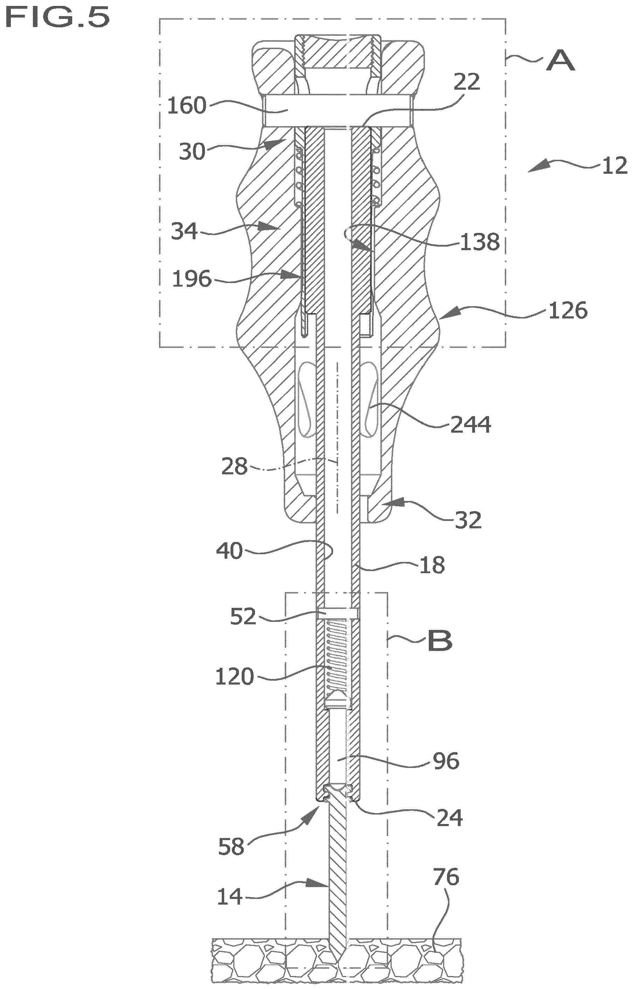

- Figur 5:: eine Schnittansicht längs Linie 5-5 in Figur 1;
- Figur 6:: eine vergrößerte Ansicht des Bereichs A in Figur 5;
- Figur 7:: eine vergrößerte Ansicht des Bereichs B in Figur 5;
- Figur 8:: eine Ansicht ähnlich Figur 5, jedoch beim Überführen des Schlaginstruments von der Fixierstellung in die Freigabestellung;
- Figur 9:: eine schematische Darstellung ähnlich Figur 8, jedoch beim Bewegen des Schlagkörpers relativ zum Schaft in distaler beziehungsweise proximaler Richtung;
- Figur 10:: eine vergrößerte Ansicht des Bereichs C in Figur 8;
- Figur 11:: eine Ansicht ähnlich Figur 5, wobei der Schlagkörper in einer beliebigen Feststellposition zwischen der distalen und der proximalen Anschlagposition am Schaft klemmend gehalten ist;
- Figur 12:: eine Ansicht ähnlich Figur 5, jedoch mit dem Schlagkörper in der proximalen Anschlagposition in der Fixierstellung;
- Figur 13:: eine Ansicht ähnlich Figur 8, also mit dem Schlaginstrument in der Schlagstellung und dem Schlagkörper in der proximalen Anschlagposition beim Ausüben eines Schlagimpulses in proximaler Richtung zum Herausziehen des medizinischen Objekt aus einem Knochen; und
- Figur 14:: eine Ansicht ähnlich Figur 8, wobei das Schlaginstrument die Schlagstellung einnimmt vor dem Ausüben eines Schlagimpulses in proximaler Richtung.

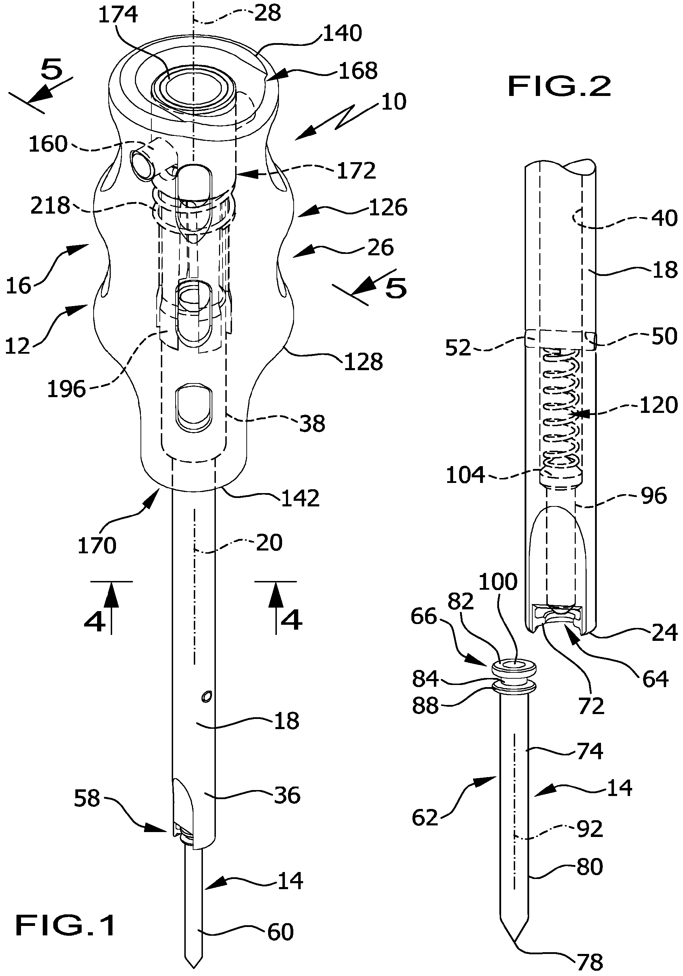

In Figur 1 ist ein Ausführungsbeispiel eines insgesamt mit dem Bezugszeichen 10 bezeichneten medizinischen Systems schematisch dargestellt. Das medizinische System 10 umfasst ein medizinisches Schlaginstrument 12 und ein medizinisches Objekt 14, welche nachfolgend noch im Einzelnen beschrieben werden.

Das in den Figuren dargestellte Ausführungsbeispiel des medizinischen Schlaginstruments 12 ist in Form eines Schlaghammers 16 ausgebildet. Dieser umfasst einen Schaft 18, welcher eine Schaftlängsrichtung 20 sowie ein proximales Ende 22 und ein distales Ende 24 definiert. Am Schaft 18 ist ein Schlagkörper 26 angeordnet, welcher eine Längsachse 28 definiert.

Der Schlagkörper 26 ist in einer Schlagstellung am Schaft 18 bewegbar, nämlich verschiebbar, und zwar zwischen einer distalen Anschlagposition, welche ein distaler Anschlag 30 definiert, und einer proximalen Anschlagposition, welche ein proximaler Anschlag 32 definiert. Diese Anordnung ermöglicht das Übertragen eines Schlagimpulses auf das distale Ende 24 des Schafts 18 in distaler sowie in proximaler Richtung.

Das Schlaginstrument 12 umfasst eine Feststelleinrichtung, welche insgesamt mit dem Bezugszeichen 34 bezeichnet ist. Sie dient zum temporären Feststellen des Schlagkörpers 26 am Schaft 18 in einer beliebigen Feststellposition zwischen der distalen Anschlagposition und der proximalen Anschlagposition.

Der Schaft 18 umfasst einen distalen Schaftabschnitt 36 und einen proximalen Schaftabschnitt 38.

Der Schaft 18 ist vom proximalen Ende 22 bis zum distalen Ende 24 durch einen Längskanal 40 durchsetzt.

Ein Außendurchmesser des proximalen Schaftabschnitts 38 ist größer als ein Außendurchmesser des distalen Schaftabschnitts 36, sodass im Übergang zwischen dem proximalen Schaftabschnitt 38 und dem distalen Schaftabschnitt 36 eine in distaler Richtung weisende Ringfläche 42 ausgebildet wird.

Der proximale Schaftabschnitt 38 umfasst den distalen Anschlag 30 mit einer in proximaler Richtung wirkenden ersten Anschlagfläche 44, welche eine proximale Endfläche 46 des Schafts 18 bildet. Die erste Anschlagfläche 44 ist ringförmig ausgebildet.

Der proximale Schaftabschnitt 38 umfasst auch den proximalen Anschlag 32 mit einer in distaler Richtung wirkenden zweiten Anschlagfläche 48, welche durch die Ringfläche 42 gebildet wird.

Am distalen Schaftabschnitt 36 ist eine Querbohrung 50 ausgebildet, in welche ein Stützelement 52 in Form eines zylindrischen Stifts 54 eingesetzt ist. Das Stützelement 52 definiert eine Stützelementlängsachse 56, welche quer, bei dem in den Figuren dargestellten Ausführungsbeispiel senkrecht, zur Schaftlängsrichtung 20 verläuft. Ein Abstand der Querbohrung vom distalen Ende 24 entspricht etwa einem Abstand der Ringfläche 42 vom proximalen Ende 22 des Schafts 18.

Am Schlaginstrument 12, nämlich an dessen Schaft 18, ist eine Kopplungseinrichtung 58 vorgesehen zum temporären Koppeln mit dem medizinischen Objekt 14.

In den Figuren ist das medizinische Objekt 14 als ein mögliches Ausführungsbeispiel in Form eines medizinischen Implantats 60 ausgebildet. Ferner ist in den Figuren das medizinische Implantat 60 in Form eines medizinischen Befestigungselements 62 beispielhaft dargestellt. Alternativ kann das medizinische Objekt 14 auch in Form einer Implantatkomponente eines Gelenkimplantats ausgebildet sein. Denkbar sind hier unterschiedliche Arten von in Knochenkavitäten einsetzbare Schäfte oder auch Gelenkpfannen von Kugelgelenken.

Die Kopplungseinrichtung 58 umfasst ein erstes Kopplungselement 64, welches nahe dem distalen Ende 24 des Schafts 18 ausgebildet ist. Das erste Kopplungselement 64 ist ausgebildet zum kraft- und/oder formschlüssigen in Eingriff Bringen mit einem korrespondierenden zweiten Kopplungselement 66 in einer Kopplungsstellung. Das medizinische Objekt 14 umfasst bei dem in den Figuren dargestellten medizinischen System 10 das zweite Kopplungselement 66.

Das erste Kopplungselement 64 umfasst eine Kopplungsaufnahme 68 in Form einer die Längsachse 28 konzentrisch umgebenden und auf diese hin weisend geöffnete Ringnut 70. An die Ringnut 70 schließt sich distalseitig ein in Richtung auf die Längsachse 28 hin vorstehender Ringflansch an.

Das medizinische Befestigungselement 62 ist in Form eines Knochenpins 74 ausgebildet, welcher zum Eintreiben in einen Knochen 76 eines Patienten an seinem distalen Ende eine Spitze 78 aufweist.

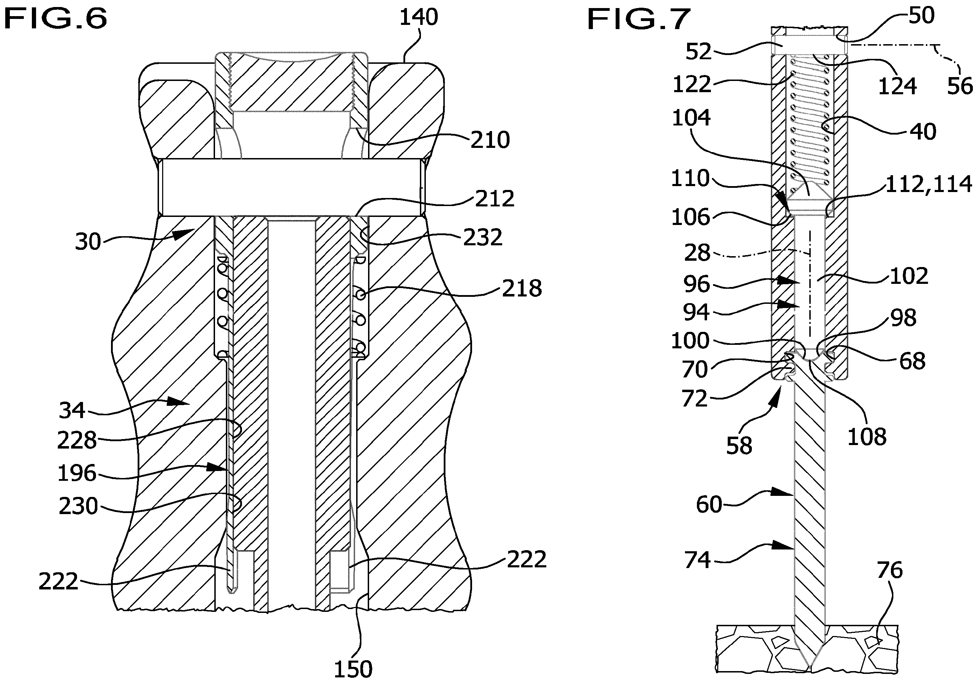

Das medizinische Befestigungselement 62 umfasst einen zylindrischen Schaft 80, welcher sich von der Spitze 78 bis zu einem proximalen Ende des medizinischen Befestigungselements 62 erstreckt, an welchem das zweite Kopplungselement 66 in Form eines Kopfs 82 ausgebildet ist. Am Kopf 82 ist eine von der Längsachse 28 weg weisende Ringnut 84 ausgebildet, in welche der Ringflansch 72 in der Kopplungsstellung eingreift, wie dies beispielhaft in Figur 7 dargestellt ist.

Der distale Schaftabschnitt 36 ist ausgehend vom distalen Ende 24 einseitig mit einer Abschrägung 86 versehen, sodass die Kopplungsaufnahme 68 seitlich geöffnet ist. Dies ermöglicht ein laterales Einschieben des Kopfs 82 in die Kopplungsaufnahme 68.

Durch die Ausbildung der Ringnut 84 wird ein zweiter Ringflansch 88 ausgebildet, welcher den Kopf 82 distalseitig begrenzt.

Der Kopf 82 und das erste Kopplungselement 64 sind derart ausgebildet, dass sie in einer Eingriffsrichtung 90 miteinander in Eingriff bringbar sind. Die Eingriffsrichtung 90 verläuft quer, bei dem in den Figuren dargestellten Ausführungsbeispiel senkrecht, zur Schaftlängsrichtung 20. Zum Koppeln des Schlaginstruments 12 und des medizinischen Objekts 14 müssen eine vom Schaft 80 des Knochenpins 74 definierte Schaftachse 92 und die Längsachse 28 parallel zueinander ausgerichtet sein.

Figur 1 zeigt die Kopplungsstellung des Schlaginstruments 12 und des medizinischen Objekts 14. In Figur 2 ist der Knochenpin 74 vor dem Koppeln mit dem ersten Kopplungselement 64 der Kopplungseinrichtung 58 schematisch dargestellt.

Die Kopplungseinrichtung 58 umfasst eine Sicherungseinrichtung 94 zum kraft- und/oder formschlüssigen Sichern des medizinischen Objekts 14 am medizinischen Schlaginstrument 12 in der Kopplungsstellung.

Die Sicherungseinrichtung 94 ist derart ausgebildet, dass sie von einer Freigabestellung, in welcher das medizinische Schlaginstrument 12 und das medizinische Objekt 14 in der Eingriffsrichtung 90 in Eingriff bringbar sind, in eine Sicherungsstellung bringbar ist, in welcher eine Bewegung des medizinischen Schlaginstruments 12 und des medizinischen Objekts 14 relativ zueinander in oder entgegengesetzt zur Eingriffsrichtung 90 blockiert ist.

Die Sicherungseinrichtung 94 umfasst ein Sicherungsglied 96, welches mit einem Sicherungselement 98 des medizinischen Objekts 14 in der Sicherungsstellung kraft- und/oder formschlüssig in Eingriff steht. Das Sicherungselement 98 ist in Form einer Vertiefung 100 am Kopf 82 ausgebildet. Die Vertiefung 100 ist in proximaler Richtung weisend geöffnet.

Das Sicherungsglied 94 umfasst einen zylindrischen Sicherungsgliedschaft 102 mit einem proximalseitig an diesem angeordneten oder ausgebildeten Sicherungsgliedkopf 104. Der Sicherungsgliedkopf 104 bildet ein proximales Ende des Sicherungsglied 96.

Der Sicherungsgliedkopf 104 definiert einen Außendurchmesser, welcher größer ist als der Außendurchmesser des Sicherungsgliedschafts 102. Dadurch wird im Übergangsbereich zwischen dem Sicherungsgliedkopf 104 und dem Sicherungsgliedschaft 102 eine Sicherungsgliedkopfanschlagfläche 106 ausgebildet, welche in distaler Richtung weist.

Ein distales Sicherungsgliedende 108 des Sicherungsglieds 96 ist mit dem medizinischen Objekt 14 in Eingriff bringbar, nämlich mit der Vertiefung 100. Das distale Sicherungsgliedende 108 ist in distaler Richtung weisend abgerundet. Eine Kontur des distalen Sicherungsgliedendes 108 und der Vertiefung 100 korrespondieren zueinander, wie dies schematisch in Figur 7 dargestellt ist. Figur 7 zeigt die Sicherungsstellung.

Zwischen dem distalen Ende 24 des Schafts 18 und der Querbohrung 50 erweitert sich ein Innendurchmesser des Längskanals 40 einstufig, sodass ein Sicherungsgliedanschlag 110 ausgebildet wird. Der Sicherungsgliedanschlag 110 umfasst eine in proximaler Richtung weisende Anschlagfläche 112, welche ringförmig ausgebildet ist. Ein Innendurchmesser des Längskanals 40 distalseitig der Anschlagfläche 112 korrespondiert zu einem Außendurchmesser des Sicherungsgliedschafts 102. Das Sicherungsglied 96 ist auf diese Weise im Längskanal 40 des Schafts 18 verschiebbar geführt gehalten.

Der Sicherungsgliedanschlag 110 der Sicherungseinrichtung 94 begrenzt eine Bewegung des Sicherungsglieds 96 am Schaft 18 in distaler Richtung. Das Sicherungsglied 96 schlägt mit seinem Sicherungsgliedkopf 104 in der Sicherungsstellung am Sicherungsgliedanschlag 110 an. Mithin sind also der Sicherungsgliedkopf 104 und der Sicherungsgliedanschlag 110 zusammenwirkend ausgebildet. In der Sicherungsstellung liegt die Sicherungsgliedkopfanschlagfläche 106 an der Anschlagfläche 112 an. Die Anschlagfläche 112 ist in Form einer Ringfläche 114 ausgebildet.

Die Sicherungseinrichtung 94 umfasst eine Vorspanneinrichtung 116 zum automatischen Überführen der Sicherungseinrichtung 94 von der Freigabestellung in die Sicherungsstellung. Die Vorspanneinrichtung 116 hält das Sicherungsglied 96 bei dem in den Figuren dargestellten Ausführungsbeispiel in der Sicherungsstellung unter Vorspannung. Hierfür umfasst die Vorspanneinrichtung 116 ein vorspannendes Element 118. Das vorspannende Element 118 ist als federelastisches Element 120 ausgebildet. Bei alternativen Ausführungsbeispielen kann das vorspannende Element in Form eines gummielastischen Elements ausgebildet sein.

Das vorspannende Element 118 ist in Form einer Druckfeder 122 ausgebildet.

Ferner stützt sich das vorspannende Element 118 einerseits distalseitig am Sicherungsglied 96, nämlich an dessen in proximaler Richtung weisenden, sich konisch verjüngenden Sicherungsgliedkopf 104, und andererseits an einer in distaler Richtung wirkenden Stützfläche 124 ab. Die Sicherungseinrichtung 94 umfasst zudem das am Schaft 18 angeordnete Stützelement 52. Das Stützelement 52 wiederum umfasst die Stützfläche 124. Es handelt sich dabei um eine äußere, in distaler Richtung weisende Außenfläche des Stützelements 52.

Die beschriebene Sicherungseinrichtung 94 ist derart ausgebildet, dass die Druckfeder 122 das Sicherungsglied 96 in distaler Richtung unter Vorspannung hält.

Wird der Kopf 82 des Knochenpins 74 ausgehend von der schematisch in Figur 2 dargestellten Trennstellung, in welcher das medizinische Objekt 14 und das medizinische Schlaginstrument 12 vollständig außer Eingriff stehen, in der Eingriffsrichtung 90, also quer zur Längsachse 28 in die Kopplungsaufnahme 68 eingeführt, gleitet das abgerundete distale Sicherungsgliedende 108 am Kopf 82 auf und wird durch diesen entgegen der Wirkung des vorspannenden Elements 118 in proximaler Richtung bewegt. Ist der Kopf 82 vollständig vom ersten Kopplungselement 64 aufgenommen, kann die Druckfeder 122 das Sicherungsglied 96 wieder in distaler Richtung bewegen, sodass das distale Sicherungsgliedende 108 in die Vertiefung 100 eintauchen und so den Knochenpin 74 gegen ein unbeabsichtigtes Entkoppeln vom medizinischen Schlaginstrument 12 sichern kann. Die beschriebene Sicherungseinrichtung 94 ist somit nach Art einer Rast- oder Schnappverbindungseinrichtung ausgebildet.

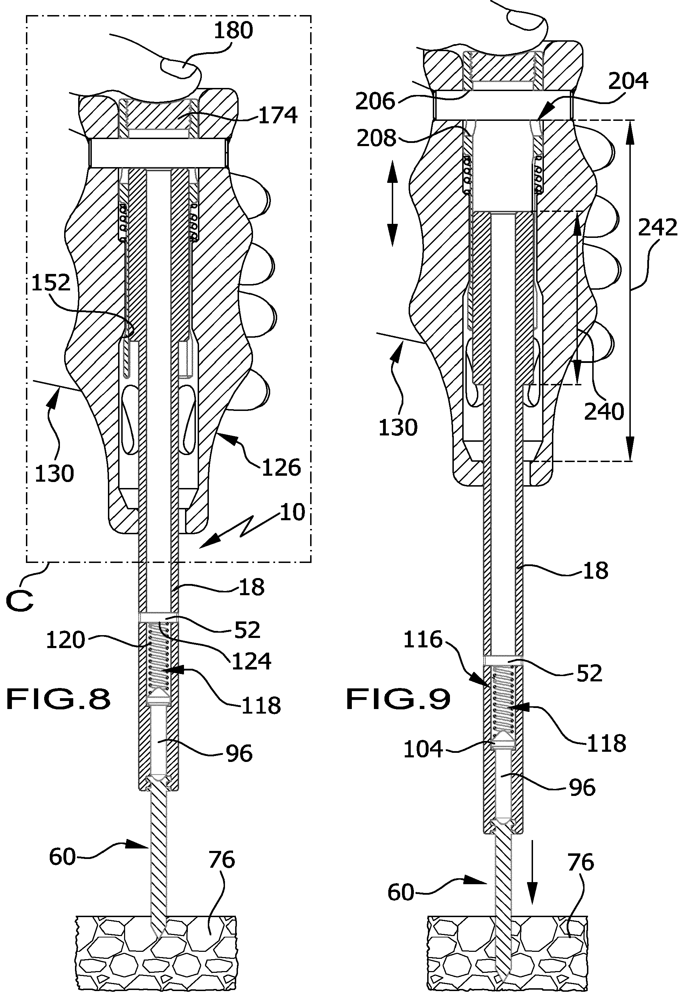

Der Schlagkörper 26 ist in Form eines Handgriffs 126 ausgebildet, welcher eine ergonomisch geformte Außenkontur 128 zum Halten desselben mit einer Hand 130 eines Anwenders ermöglicht. Die ergonomische Außenkontur 128 umfasst zwei voneinander beanstandete Ringvorsprünge 132 und 134, zwischen denen eine Ringnut 136 ausgebildet ist. Proximalseitig des Ringvorsprungs 132 und distalseitig des Ringvorsprungs 134 verringert sich ein Außendurchmesser des Handgriffs 126 in proximaler beziehungsweise distaler Richtung. Die Außenkontur 128 ist vollständig verrundet und ohne scharfe Ecken und Kanten ausgebildet. In den Figuren 8 bis 10 sowie 13 und 14 ist schematisch dargestellt, wie das medizinische Schlaginstrument 12 von einem Anwender mit einer Hand gehalten werden kann.

Der Schlagkörper 26 weist eine sich koaxial zur Schaftlängsrichtung 20 erstreckende Längsdurchbrechung 138 auf. Der Schaft 18 ist mindestens teilweise in der Längsdurchbrechung 138 aufgenommen.

Der Schlagkörper 26 umfasst eine in proximaler Richtung weisende, ringförmige proximale Schlagkörperendfläche 140 und eine in distaler Richtung weisende distale Schlagkörperendfläche 142.

Der im Wesentlichen hülsenförmige Schlagkörper 26 umfasst eine die Längsachse 28 umgebende Umfangswand 144 und eine distale Begrenzungswand 146.

Die Längsdurchbrechung 138 durchsetzt die distale Begrenzungswand 146. Ein Innendurchmesser der Längsdurchbrechung 138 im Bereich der distalen Begrenzungswand 146 korrespondiert zum Außendurchmesser des distalen Schaftabschnitts 36. Auf diese Weise wird im Bereich der distalen Begrenzungswand 146 eine in Richtung auf die Längsachse hin weisende Führungsfläche 148 für den distalen Schaftabschnitt 36 gebildet.

Proximalseitig der distalen Begrenzungswand erweitert sich ein Innendurchmesser der Längsdurchbrechung 138 zunächst konisch und bleibt dann etwa auf einem Drittel einer Gesamtlänge des Schlagkörpers 26 konstant. So wird eine Ausnehmung 150 ausgebildet, welche einen Innendurchmesser aufweist, der größer ist sowohl als ein Außendurchmesser des distalen Schaftabschnitts 36 als auch größer als ein Außendurchmesser des proximalen Schaftabschnitts 38. Ein Innendurchmesser der Ausnehmung 150 verjüngt sich proximalseitig konisch und bildet so eine konische Klemmfläche 152. Die Klemmfläche 152 begrenzt die Ausnehmung 150 abschnittsweise mit der in Richtung auf die Längsachse 28 hin weisenden Klemmfläche 152.

An die Klemmfläche 152 schließt sich ein hohlzylindrischer Abschnitt 154 der Längsdurchbrechung 138 an. Ein Innendurchmesser des Abschnitts 154 ist etwas größer als ein Außendurchmesser des proximalen Schaftabschnitts 38. Der Grund hierfür wird nachfolgend noch erläutert.

Proximalseitig erweitert sich ein Innendurchmesser der Längsdurchbrechung 138 an den Abschnitt 154 anschließend einstufig, so dass eine in proximaler Richtung weisende Schlagkörperstützfläche 156 ausgebildet wird. Die Schlagkörperstützfläche 156 ist in Form einer Ringfläche 158 ausgebildet. Ein Innendurchmesser der Längsdurchbrechung 138 ausgehend von der Ringfläche 158 bis zur proximalen Schlagkörperendfläche 140 entspricht etwa einem Innendurchmesser der Ausnehmung 150.

Am Schlagkörper 26 ist ferner ein Anschlagelement 160 angeordnet. Es definiert eine Anschlagelementlängsachse 162, welcher quer, bei dem in den Figuren dargestellten Ausführungsbeispiel senkrecht, zur Schaftlängsrichtung 20 verläuft. Das Anschlagelement 160 ist in Form eines in eine Querbohrung 164 am Schlagkörper 26 eingesetzten Anschlagstifts 166 ausgebildet.

Die Querbohrung 164 ist proximalseitig der Ringfläche 158 ausgebildet, und zwar näher zur proximalen Schlagkörperendfläche 140 hin als zur Ringfläche 158. Somit ist auch ein Abstand des Anschlagelements 160 von der proximalen Schlagkörperendfläche 140, welche ein proximales Ende 168 des Schlagkörpers 26 definiert, kleiner als ein Abstand des Anschlagelements 160 von der distalen Schlagkörperendfläche 142, welche ein distales Ende 170 des Schlagkörpers 26 definiert.

Im Folgenden sollen nun der Aufbau und die Funktionsweise der Feststelleinrichtung 34 näher definiert werden. Sie umfasst bei dem in den Figuren dargestellten Ausführungsbeispiel ein Feststellelement 172 zum kraft- und/oder formschlüssigen in Eingriff Bringen sowohl mit dem Schaft 18 als auch mit dem Schlagkörper 26 in einer der oben beschriebenen beliebigen Feststellpositionen zwischen der distalen Anschlagposition und der proximalen Anschlagposition.

Das Feststellelement 172 ist hülsenförmig ausgebildet. Ein Innendurchmesser des Feststellelements 172 ist korrespondierend zum Außendurchmesser des proximalen Schaftabschnitts 38 ausgebildet, sodass das Feststellelement 172 und der proximale Schaftabschnitt 38 in der Schlagstellung parallel zur Schaftlängsrichtung 20 verschiebbar sind. Das Feststellelement 172 ist so auch am proximalen Schaftabschnitt 38 geführt.

Proximalseitig ist das Feststellelement 172 verschlossen. Ein Verschlusselement 174 ist ausgehend von einem proximalen Ende 176 des Feststellelements 172 in dieses eingeschraubt. Das Verschlusselement 174 weist eine flache Vertiefung 178 auf, um beispielsweise einen Daumen 180 eines Anwenders teilweise aufzunehmen und dessen Abrutschen zu verhindern.

Das Feststellelement 172 umfasst einen Hülsenabschnitt 182. Er erstreckt sich ausgehend vom proximalen Ende 176 in distaler Richtung. Von einer in distaler Richtung weisenden Endfläche 184 des Hülsenabschnitts steht ein im Außendurchmesser verringerter hülsenförmiger Klemmelementabschnitt 186 ab, sodass ein Teil der Endfläche 184 eine Feststellelementstützfläche 188 bildet. Diese ist ringförmig und umgibt den Klemmelementabschnitt 186.

Zu seinem distalen Ende 190 hin erweitert sich ein Außendurchmesser des Klemmelementabschnitt 186 konisch und bildet eine Aufgleitfläche 192. An die Aufgleitfläche 192 schließt sich ein zylindrischer Endabschnitt 194 des Klemmelementabschnitts 186 an. Der Endabschnitt 194 definiert einen Außendurchmesser, welcher größer ist als ein Außendurchmesser des Klemmelementabschnitts 186 proximalseitig der Aufgleitfläche 192. Der Außendurchmesser des Endabschnitts 194 ist jedoch kleiner als ein Außendurchmesser des Hülsenabschnitts 182.

Das Feststellelement 172 ist in Form eines Klemmelements 196 ausgebildet. Ausgehend vom distalen Ende 190 sind parallel zur Längsachse 28 mehrere Schlitze 198 ausgebildet, sodass eine entsprechende Zahl von Klemmarmen 200 ausgebildet wird. Bei dem in den Figuren dargestellten Ausführungsbeispiel sind sechs Schlitze 198 vorgesehen. Die Schlitze 198 erstrecken sich bis an den Hülsenabschnitt 182 heran. Die Klemmarme 200 weisen aufgrund der Ausgestaltung des Endabschnitts 194 eine Verdickung an ihren freien Enden 222 auf. Zudem ist an jedem Klemmarm 200 ein verbleibender Abschnitt der Aufgleitfläche 192 ausgebildet.

Der Klemmelementabschnitt 186 weist einen Außendurchmesser auf, welcher zum Innendurchmesser des Abschnitts 154 des Schlagkörpers 26 korrespondiert. Mithin ist also das Feststellelement 172 am Schlagkörper 26 im Bereich des Abschnitts 154 mit einer Außenfläche des Klemmelementabschnitts 186 geführt.

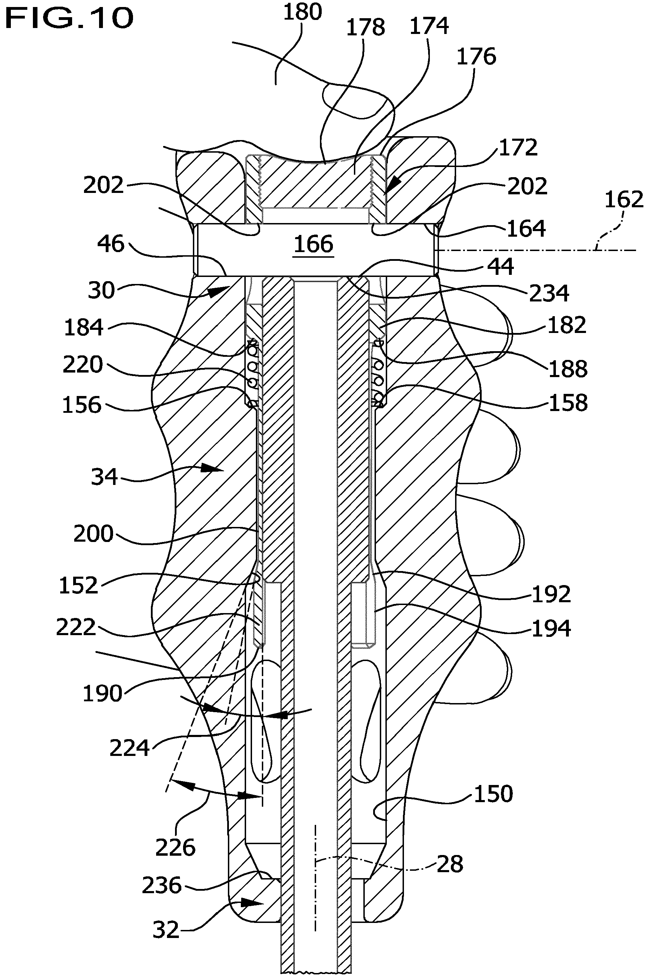

Ferner ist am Feststellelement 172 eine Feststellelementdurchbrechung 202 ausgebildet. Diese erstreckt sich quer, bei dem in den Figuren dargestellten Ausführungsbeispiel senkrecht, zur Längsachse 28. Die Feststellelementdurchbrechung 202 ist im Bereich des Hülsenabschnitts 182 ausgebildet und langlochartig parallel zur Längsachse 28 geformt. Das Anschlagelement 160 durchsetzt die Feststellelementdurchbrechung 202, wie dies beispielsweise in Figur 10 gut zu erkennen ist. Ein Außendurchmesser des Anschlagstifts 166 ist kleiner als eine Längserstreckung der Feststellelementdurchbrechung 202 parallel zur Längsachse 28, sodass das Feststellelement 172 parallel zur Längsachse 28 bewegbar ist.

Der Schlagkörper 26 umfasst ferner eine Feststellelementanschlageinrichtung 204. Die Feststellelementanschlageinrichtung 204 umfasst einen distalen Feststellelementanschlag 206 und einen proximalen Feststellelementanschlag 208 zum Begrenzen einer Bewegung des Feststellelements 172 in distaler und proximaler Richtung. Bei dem in den Figuren dargestellten Ausführungsbeispiel ist der distale Feststellelementanschlag 206 am Feststellelement 172 proximalseitig bezogen auf den proximalen Feststellelementanschlag 208 angeordnet beziehungsweise ausgebildet. Der distale Feststellelementanschlag 206 wird durch eine in distaler Richtung weisende innere Endfläche der Feststellelementdurchbrechung 202 gebildet. Der proximale Feststellelementanschlag 208 wird durch eine in proximaler Richtung weisende innere Endfläche 212 der Feststellelementdurchbrechung 202 gebildet.

Die Feststelleinrichtung 34 umfasst ferner eine Rückstelleinrichtung 214. Sie dient dem Zweck, die Feststelleinrichtung 34 in einer Fixierstellung zu halten.

In der Fixierstellung ist der Schlagkörper 26 in einer beliebigen Feststellposition am Schaft 18 festgelegt. Die Feststelleinrichtung 34 ist ferner derart ausgebildet, dass sie von der Fixierstellung in die Schlagstellung, in welcher der Schlagkörper 26 und der Schaft 18 relativ zueinander bewegbar sind, überführbar ist und umgekehrt. Bei dem in den Figuren dargestellten Ausführungsbeispiel ist die Feststelleinrichtung 34 derart ausgebildet, dass sie entgegen der Wirkung der Rückstelleinrichtung 214 von der Fixierstellung in die Schlagstellung bringbar ist. Mithin kann bei dem in den Figuren dargestellten Ausführungsbeispiel das Feststellelement 172 entgegen der Wirkung der Rückstelleinrichtung 214 bewegt werden, um die Feststelleinrichtung 34 von der Fixierstellung in die Schlagstellung zu überführen.

Die Rückstelleinrichtung 214 umfasst ein Rückstellelement 216. Das Rückstellelement 216 ist bei dem in den Figuren dargestellten Ausführungsbeispiel in Form eines federelastischen Elements 218 ausgebildet. Bei alternativen Ausführungsbeispielen ist das Rückstellelement in Form eines oder mehrerer gummielastischer Elemente ausgebildet.

Das Rückstellelement 216 ist bei dem in den Figuren dargestellten Ausführungsbeispiel in Form einer Druckfeder 220 ausgebildet.

Das Rückstellelement 216 stützt sich einerseits an der Feststellelementstützfläche 188 des Feststellelements 172 und andererseits an der Schlagkörperstützfläche 156 des Schlagkörpers 26 ab. Die Feststellelementstützfläche 188 weist in distaler Richtung und ist wie erwähnt als Ringfläche ausgebildet. Sie bildet einen Teil der Endfläche 184.

Das Rückstellelement 216 ist das Feststellelement 172 umgebend angeordnet beziehungsweise ausgebildet. Das Rückstellelement 216 umgibt das Feststellelement 172 im Bereich des Klemmabschnitts 186. Wie beschrieben stützt es sich proximalseitig am Hülsenabschnitt 182, nämlich dessen Endfläche 184, ab.

Das Feststellelement 172 ist zum Überführen der Feststelleinrichtung 34 von der Fixierstellung in die Schlagstellung relativ zum Schlagkörper bewegbar, nämlich verschiebbar. Bei dem in den Figuren dargestellten Ausführungsbeispiel ist das Feststellelement 172 zum Überführen der Feststelleinrichtung 34 von der Fixierstellung in die Schlagstellung relativ zum Schlagkörper 26 in distaler Richtung bewegbar.

Die Funktionsweise der Feststelleinrichtung 34 wird nachfolgend erläutert.

Figur 6 zeigt die Fixierstellung. Das Rückstellelement 216 drückt das Feststellelement 172 mit der distalen Endfläche 212 gegen das Anschlagelement, wodurch eine Bewegung des Feststellelements 172 in proximaler Richtung begrenzt ist. In dieser Stellung sind die Endabschnitte 194 der Klemmarme 200 in den Bereich des Abschnitts 154 zurückgezogen und wirken kraft- und/oder formschlüssig sowohl mit dem Schaft 18 als auch mit dem Schlagkörper 26 zusammen. Der Schlagkörper 26 ist in dieser Fixierstellung klemmend am Schaft 18 gehalten.

Um den Schlagkörper 26 relativ zum Schaft 28 bewegen zu können, muss die Feststelleinrichtung 34 von der Fixierstellung in die Schlagstellung überführt werden. Hierzu kann ein Anwender, beispielsweise mit seinem Daumen 180, auf das Verschlusselement 174 am Feststellelement 172 drücken und so das Feststellelement 172 entgegen der Wirkung der Rückstelleinrichtung 214 in distaler Richtung bewegen, bis die proximale Endfläche 210 der Feststellelementdurchbrechung 202 am Anschlagelement 160 anschlägt. Bei dieser Bewegung des Feststellelements 172 relativ zum Schlagkörper 26 in distaler Richtung bewegen sich die Endabschnitte 194 in den Bereich der Ausnehmung 150. Durch den größeren Innendurchmesser der Ausnehmung 150 ist es nun möglich, dass die freien Enden 222 der Klemmarme 200 von der Längsachse 82 des Schafts 18 weg verschwenkbar sind. Oder anders ausgedrückt werden die in distaler Richtung verschobenen Klemmarme 200 nicht mehr durch die Klemmfläche 152 beziehungsweise die innere Wandfläche des Abschnitts 154 gegen den proximalen Schaftabschnitt 38 gespannt. In der Schlagstellung, wie sie beispielshaft in den Figuren 8 bis 10 sowie 13 und 14 schematisch dargestellt ist, ist der Schlagkörper 26 relativ zum Schaft 18 parallel zur Längsachse 28 verschiebbar.

In der Fixierstellung verhindern die Klemmfläche 152 beziehungsweise die innere Wandfläche des Abschnitts 154 eine Bewegung der freien Enden 222 der Klemmarme 200 beziehungsweise des Klemmelements 196 eine Bewegung von der Längsachse 28 weg, sodass wie beschrieben das Klemmelement 196 in Richtung auf die Längsachse 28 in der Fixierstellung hin gehalten wird, um den Schlagkörper 26 am Schaft 18 in einer beliebigen Feststellposition festzustellen, nämlich zu klemmen.

Ferner sei angemerkt, dass ein erster, zwischen der Klemmfläche 152 und der Längsachse 28 definierter Neigungswinkel 224 größer ist als ein zweiter, zwischen der Aufgleitfläche 192 und der Längsachse 28 definierter Neigungswinkel 226.

Durch die beschriebene Ausgestaltung des medizinischen Schlaginstruments 12 bildet eine in Richtung auf die Längsachse 28 hin weisende Innenfläche 228 des Feststellelements 172 eine Führungsfläche 230 für den proximalen Schaftabschnitt 38.

Ferner bildet die Längsdurchbrechung 138 des Schlagkörpers 26 von dessen proximalem Ende eine in Richtung auf die Längsachse 28 hin weisende Führungsfläche 232 für das Feststellelement 172, nämlich eine Außenfläche des Hülsenabschnitts 182.

Ist die Feststelleinrichtung 34 von der Fixierstellung in die Schlagstellung überführt, lässt sich der Schlagkörper 26 relativ zum Schaft 18 bewegen. Der proximale Schaftabschnitt 38 umfasst dabei wie bereits erwähnt den distalen Anschlag 30 mit der in proximaler Richtung wirkenden ersten Anschlagfläche 44.

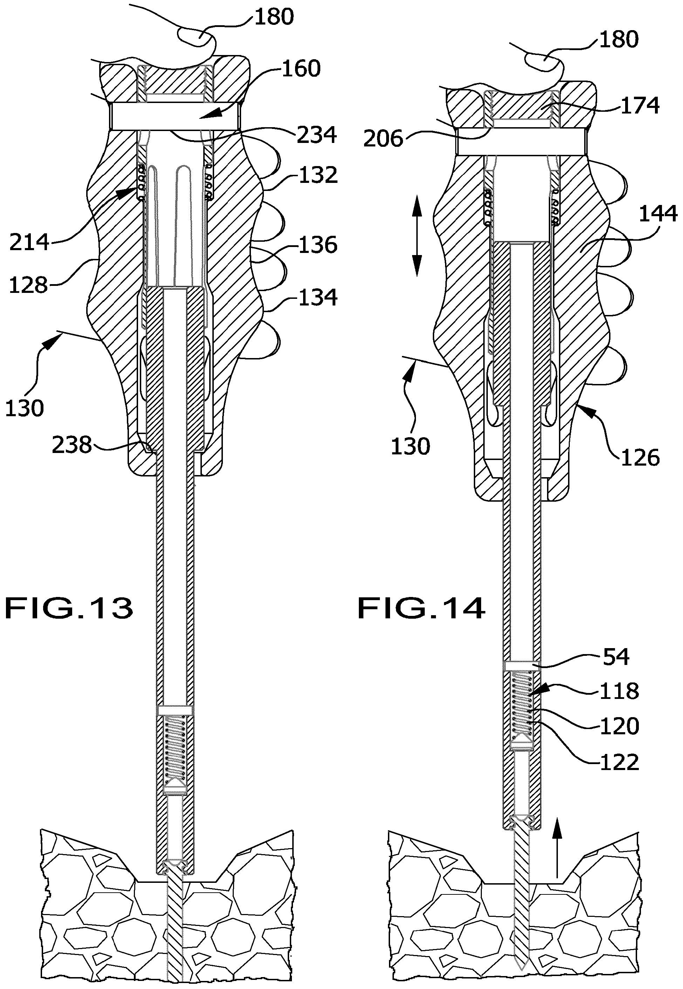

Der Schlagkörper 26 umfasst eine mit der ersten Anschlagfläche 44 zusammenwirkende erste Schlagkörperanschlagfläche 234, welche in distaler Richtung wirkt. Bei dem in den Figuren dargestellten Ausführungsbeispiel definiert das Anschlagelement 160 die erste Schlagkörperanschlagfläche 234. Dies bedeutet, dass in der Schlagstellung der Schlagkörper 26 nur so weit in distaler Richtung bewegt werden kann, bis das Anschlagelement 160 mit der Schlagkörperanschlagfläche 234 an der ersten Anschlagfläche 44, mithin also an der proximalen Endfläche 46 des Schafts 18 anschlägt. Ausgehend von dieser Extremstellung kann der Schlagkörper 26 relativ zum Schaft 18 in der Schlagstellung in proximaler Richtung bewegt werden, bis eine zweite Schlagkörperanschlagfläche 236 des Schlagkörpers 26 an der zweiten Anschlagfläche 48, mithin also an der Ringfläche 42, anschlägt, wie dies beispielhaft in Figur 13 schematisch dargestellt ist.

Die zweite Schlagkörperanschlagfläche 236 ist ebenfalls in Form einer Ringfläche 238 ausgebildet, welche in proximaler Richtung weist. Sie bildet eine Seitenfläche der distalen Begrenzungswand 146. Somit umfasst die distale Begrenzungswand 146 die zweite Schlagkörperanschlagfläche 236.

Ein erster Abstand 240 zwischen der ersten Anschlagfläche 44 und der zweiten Anschlagfläche 48 ist kleiner als ein zweiter Abstand 242 zwischen der ersten Schlagköperanschlagfläche 234 und der zweiten Schlagkörperanschlagfläche 236. Eine Differenz der Abstände 240 und 242 gibt einen maximalen Bewegungshub des Schlagkörper 26 relativ zum Schaft 18 vor.

Um mit dem medizinischen Schlaginstrument 12 auch Kraftimpulse auf dessen distales Ende 24 in der Fixierstellung übertragen zu können, beispielsweise mit einem Hammer, welcher auf die proximale Schlagkörperendfläche 140 einwirkt, steht das proximale Ende 176 des Feststellelements 172 in der Fixierstellung nicht oder im Wesentlichen nicht über das proximale Ende 168 des Schlagkörpers 26 vor. Das medizinische Schlaginstrument 12 kann so auch als eine Art Meisel benutzt werden. Optional können so größere Kraftimpulse auf das distale Ende 24 ausgeübt werden als mit dem Schlagkörper 26, wenn dieser in der Schlagstellung mit dem Anschlagelement 160 gegen die proximale Endfläche 46 bewegt wird.

Bei dem in den Figuren dargestellten medizinischen Schlaginstrument 12 ist der Schlagkörper 26 in einer Grundstellung relativ zum Schaft 18 festgestellt und nimmt eine Feststellposition ein. Die Feststelleinrichtung 34 nimmt dabei die Fixierstellung ein. Mit der Feststelleinrichtung 34 lässt sich der Schlagkörper 26 am Schaft 18 nicht nur in einer Feststellposition zwischen der distalen Anschlagposition und der proximalen Anschlagposition feststellen, sondern auch in der proximalen Anschlagposition und in der distalen Anschlagposition.

Für eine optimale Reinigung des medizinischen Schlaginstruments 12 sind mehrere am Schlagkörper 26 ausgebildete Spülöffnungen 244 und 246 vorgesehen. Die Spülöffnungen 244 sind im Bereich der Umfangswand 144 des Schlagkörpers 26 ausgebildet, die Spülöffnungen 246 im Bereich der distalen Begrenzungswand 146 des Schlagkörpers 26. Die Spülöffnungen 246 sind als Erweiterungen der Führungsfläche 148 ausgebildet, sodass lediglich noch Abschnitte derselben verbleiben, an denen der distale Schaftabschnitt 36 beim Bewegen des Schlagkörpers 26 in der Schlagstellung relativ zum Schaft 18 geführt ist.

Die Spülöffnungen 244 schaffen einen Zugang zur Längsdurchbrechung 138.

Der Einsatz des Schlaginstruments 12 wird nachfolgend in aller Kürze in Verbindung mit den Figuren 8 bis 14 erläutert.

Um den wie oben beschrieben mit dem distalen Ende 24 gekoppelten Knochenpin 74 in den Knochen 76 des Patienten einzutreiben, wird die Feststelleinrichtung 34 in die Schlagstellung überführt durch Verschieben des Feststellelements 172 in distaler Richtung wie schematisch in Figur 8 dargestellt. Nun kann der Schlagkörper 26 mit dem Anschlagelement 160 gegen die erste Anschlagfläche 44 bewegt werden, um einen Schlagimpuls auf den Knochenpin 74 zu übertragen und diesen in den Knochen 76 einzutreiben. In der Schlagstellung kann der Schlagkörper 26 relativ zum Schaft 18 wieder in proximaler Richtung bewegt und dann erneut zum Übertragen eines Schlagimpulses in Richtung auf die erste Anschlagfläche 44 hin bewegt werden.

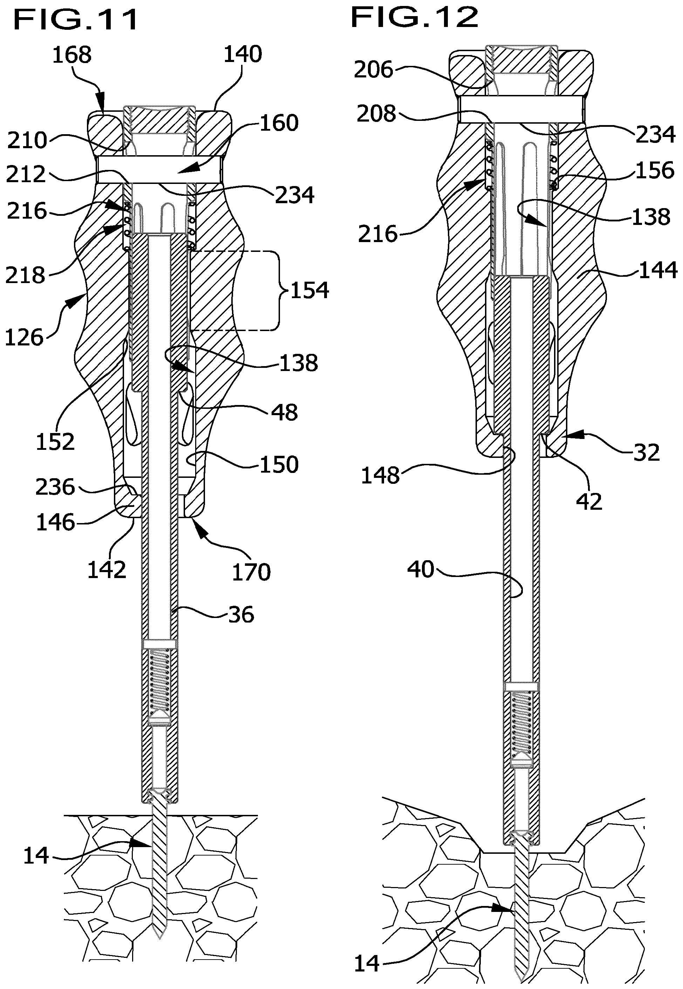

Die Feststelleinrichtung 34 ermöglicht eine beliebige Feststellposition beziehungsweise eine beliebige Klemmung des Schlagkörpers 26 zwischen den extremen Anschlagpositionen, nämlich der distalen Anschlagposition und der proximalen Anschlagposition. Eine solche Zwischenstellung, also eine Feststellposition zwischen der distalen Anschlagposition und der proximalen Anschlagposition ist beispielhaft in Figur 11 dargestellt. Die Feststelleinrichtung 34 nimmt hier die Fixierstellung ein. In Figur 12 ist die Feststelleinrichtung 34 ebenfalls in der Fixierstellung dargestellt. Allerdings nimmt der Schlagkörper 26 hier seine proximale Anschlagposition ein.

Um den Knochenpin 74 wieder aus dem Knochen 76 zu lösen, wird die Feststelleinrichtung 34 von der Fixierstellung in die Schlagstellung überführt und der Schlagkörper 26 mit der zweiten Schlagkörperanschlagfläche 236 gegen die erste Anschlagfläche 48 bewegt. So kann ein Schlagimpuls in proximaler Richtung mit dem Schlagkörper 26 auf den Schaft 18 ausgeübt werden, um den Knochenpin 74 aus dem Knochen 76 sukzessive durch wiederholtes Ausüben eines solchen Schlagimpulses herauszuziehen. Dies ist schematisch in den Figuren 13 und 14 dargestellt.

Das beschriebene medizinische Schlaginstrument 12 ermöglicht eine einfache und unkomplizierte Ankopplung desselben beispielsweise an ein in einem Knochen 76 verankertes medizinisches Objekt 14 mit einer Hand. Durch Freigeben des Feststellelements 172, sodass die Feststelleinrichtung 34 die Fixierstellung einnimmt, kann der Schlagkörper 26 in einer der beschriebenen beliebigen Feststellpositionen unbeweglich am Schaft 18 gesichert werden. Ein Anwender kann nun das distale Ende 24 des Schlaginstruments 12 an das medizinische Objekt 14 heranführen, beispielsweise an den Kopf 82 des Knochenpins 74, und diesen in der Eingriffsrichtung 90 mit dem ersten Kopplungselement 64 koppeln. Dies ist mit nur einer Hand, also ohne Zuhilfenahme einer zweiten Hand, möglich, da das distale Ende 24 in der Fixierstellung relativ zum Schlagkörper 26, den der Anwender mit seiner Hand 130 hält und führt, in der Fixierstellung feststeht.

Somit ermöglicht das beschriebene medizinische System 10 mit seinem medizinischen Schlaginstrument 12 eine verbesserte Handhabung im Vergleich mit bekannten medizinischen Schlaginstrumenten.

### Bezugszeichenliste

- 10: medizinisches System
- 12: medizinisches Schlaginstrument
- 14: medizinisches Objekt
- 16: Schlaghammer
- 18: Schaft
- 20: Schaftlängsrichtung
- 22: proximales Ende
- 24: distales Ende
- 26: Schlagkörper
- 28: Längsachse
- 30: distaler Anschlag
- 32: proximaler Anschlag
- 34: Feststelleinrichtung
- 36: distaler Schaftabschnitt
- 38: proximaler Schaftabschnitt
- 40: Längskanal
- 42: Ringfläche
- 44: erste Anschlagfläche
- 46: proximale Endfläche
- 48: zweite Anschlagfläche
- 50: Querbohrung
- 52: Stützelement
- 54: Stift
- 56: Stützelementlängsachse
- 58: Kopplungseinrichtung
- 60: medizinisches Implantat
- 62: medizinisches Befestigungselement
- 64: erstes Kopplungselement
- 66: zweites Kopplungselement
- 68: Kopplungsaufnahme
- 70: Ringnut
- 72: Ringflansch
- 74: Knochenpin
- 76: Knochen
- 78: Spitze
- 80: Schaft
- 82: Kopf
- 84: Ringnut
- 86: Abschrägung
- 88: Ringflansch
- 90: Eingriffsrichtung
- 92: Schaftachse
- 94: Sicherungseinrichtung
- 96: Sicherungsglied
- 98: Sicherungselement
- 100: Vertiefung
- 102: Sicherungsgliedschaft
- 104: Sicherungsgliedkopf
- 106: Sicherungsgliedkopfanschlagfläche
- 108: distales Sicherungsgliedende
- 110: Sicherungsgliedanschlag
- 112: Anschlagfläche
- 114: Ringfläche
- 116: Vorspanneinrichtung
- 118: vorspannendes Element
- 120: federelastisches Element
- 122: Druckfeder
- 124: Stützfläche
- 126: Handgriff
- 128: Außenkontur
- 130: Hand
- 132: Ringvorsprung
- 134: Ringvorsprung
- 136: Ringnut
- 138: Längsdurchbrechung
- 140: proximale Schlagkörperendfläche
- 142: distale Schlagkörperendfläche
- 144: Umfangswand
- 146: distale Begrenzungswand
- 148: Führungsfläche
- 150: Ausnehmung
- 152: Klemmfläche
- 154: Abschnitt
- 156: Schlagkörperstützfläche
- 158: Ringfläche
- 160: Anschlagelement
- 162: Anschlagelementlängsachse
- 164: Querbohrung
- 166: Anschlagsstift
- 168: proximales Ende
- 170: distales Ende
- 172: Feststellelement
- 174: Verschlusselement
- 176: proximales Ende
- 178: Vertiefung
- 180: Daumen
- 182: Hülsenabschnitt
- 184: Endfläche
- 186: Klemmelementabschnitt
- 188: Feststellelementstützfläche
- 190: distales Ende
- 192: Aufgleitfläche
- 194: Endabschnitt
- 196: Klemmelement
- 198: Schlitz
- 200: Klemmarm
- 202: Feststellelementdurchbrechung
- 204: Feststellelementanschlageinrichtung
- 206: distaler Feststellelementanschlag
- 208: proximaler Feststellelementanschlag
- 210: proximale Endfläche
- 212: distale Endfläche
- 214: Rückstelleinrichtung
- 216: Rückstellelement
- 218: federelastisches Element
- 220: Druckfeder
- 222: freies Ende
- 224: erster Neigungswinkel
- 226: zweiter Neigungswinkel
- 228: Innenfläche
- 230: Führungsfläche
- 232: Führungsfläche
- 234: erste Schlagkörperanschlagfläche
- 236: zweite Schlagkörperanschlagfläche
- 238: Ringfläche
- 240: erster Abstand
- 242: zweiter Abstand
- 244: Spülöffnung
- 246: Spülöffnung

## Patentansprüche

1. Medizinisches Schlaginstrument (12), insbesondere in Form eines Schlaghammers (16), mit einem eine Schaftlängsrichtung (20) sowie ein proximales und ein distales Ende (22, 24) definierenden Schaft (18) und einem am Schaft (18) angeordneten, eine Längsachse (28) definierenden Schlagkörper (26), wobei der Schlagkörper (26) in einer Schlagstellung am Schaft (18) zwischen einem eine distale Anschlagposition des Schlagkörpers (26) definierenden distalen Anschlag (30) und einem eine proximale Anschlagposition des Schlagkörpers (26) definierenden proximalen Anschlag (32) bewegbar, insbesondere verschiebbar, ist zum Übertragen eines Schlagimpulses auf das distale Ende (24) des Schafts (18) in distaler beziehungsweise proximaler Richtung, wobei das Schlaginstrument (12) eine Feststelleinrichtung (34) umfasst zum temporären Feststellen des Schlagkörpers (26) am Schaft (18) in mindestens einer, insbesondere beliebigen, Feststellposition zwischen der distalen Anschlagposition und der proximalen Anschlagposition, wobei die Feststelleinrichtung (34) von einer Fixierstellung, in welcher der Schlagkörper (26) in einer der beliebigen Feststellpositionen am Schaft (18) festgelegt ist, in die Schlagstellung, in welcher der Schlagkörper (26) und der Schaft (18) relativ zueinander bewegbar sind, überführbar ist und umgekehrt, **dadurch gekennzeichnet, dass** der Schlagkörper (26) in einer Grundstellung relativ zum Schaft (18) festgestellt ist und die mindestens eine Feststellposition einnimmt und dass die Feststelleinrichtung (34) eine Rückstelleinrichtung (214) umfasst zum automatischen Halten der Feststelleinrichtung (34) in der Fixierstellung.

2. Medizinisches Schlaginstrument nach Anspruch 1, **dadurch gekennzeichnet, dass** die Rückstelleinrichtung (214) mindestens ein Rückstellelement (216) umfasst,
wobei insbesondere das mindestens eine Rückstellelement (216) in Form eines gummi- oder federelastischen Elements (218) oder in Form einer Druck- oder Zugfeder (220) ausgebildet ist.

3. Medizinisches Schlaginstrument nach einem der voranstehenden Ansprüche, **dadurch gekennzeichnet, dass** die Feststelleinrichtung (34) mindestens ein Feststellelement (172) umfasst zum kraft- und/oder formschlüssigen in Eingriff Bringen sowohl mit dem Schaft (18) als auch mit dem Schlagkörper (26) in der mindestens einen Feststellposition.

4. Medizinisches Schlaginstrument nach Anspruch 3, **dadurch gekennzeichnet, dass** sich das mindestens eine Rückstellelement (216) einerseits an einer Feststellelementstützfläche (188) des mindestens einen Feststellelements (172) und andererseits an einer Schlagkörperstützfläche (156) des Schlagkörpers (26) abstützt,
wobei insbesondere die Feststellelementstützfläche (188) in distaler Richtung weisend ausgebildet ist, insbesondere als Ringfläche, und wobei die Schlagkörperstützfläche (156) in proximaler Richtung weisend ausgebildet ist, insbesondere als Ringfläche (158).

5. Medizinisches Schlaginstrument nach Anspruch 3 oder 4, **dadurch gekennzeichnet, dass** das mindestens eine Feststellelement (172)
a) zum Überführen der Feststelleinrichtung (34) von der Fixierstellung in die Schlagstellung relativ zum Schlagkörper (26) bewegbar, nämlich verschiebbar, ist und/oder
b) hülsenförmig ausgebildet ist
und/oder
c) zum Überführen der Feststelleinrichtung (34) von der Fixierstellung in die Schlagstellung relativ zum Schlagkörper (26) in distaler Richtung bewegbar ist
und/oder
d) proximalseitig verschlossen ist.

6. Medizinisches Schlaginstrument nach Anspruch 5, **dadurch gekennzeichnet, dass** das mindestens eine Feststellelement (172) in Form eines Klemmelements (196) ausgebildet ist und mindestens einen sich in Längsrichtung erstreckenden Klemmarm (200) umfasst und dass ein freies Ende (222) des mindestens einen Klemmarms (200) in Richtung auf die Längsachse (28) des Schafts (26) hin und von dieser weg bewegbar, insbesondere verschwenkbar, ausgebildet ist.

7. Medizinisches Schlaginstrument nach Anspruch 6, **dadurch gekennzeichnet, dass** der Schlagkörper (26) eine den Schaft (18) umgebende Ausnehmung (150) zum Aufnehmen des mindestens eine Klemmelements (196) umfasst.

8. Medizinisches Schlaginstrument nach einem der voranstehenden Ansprüche, **dadurch gekennzeichnet, dass** der Schlagkörper (26) eine sich koaxial zur Schaftlängsrichtung (20) erstreckende Längsdurchbrechung (138) aufweist und dass der Schaft (18) mindestens teilweise in der Längsdurchbrechung (138) aufgenommen ist.

9. Medizinisches Schlaginstrument nach Anspruch 8, **dadurch gekennzeichnet, dass** der Schaft (18) einen distalen Schaftabschnitt (36) und einen proximalen Schaftabschnitt (38) umfasst, dass der proximale Schaftabschnitt (38) in der Längsdurchbrechung (138) aufgenommen ist und dass der distale Schaftabschnitt (36) distalseitig aus der Längsdurchbrechung (138) vorsteht.

10. Medizinisches Schlaginstrument nach Anspruch 8 oder 9, **dadurch gekennzeichnet, dass** die Längsdurchbrechung (138) des Schlagkörpers (26) ausgehend von dessen proximalem Ende eine in Richtung auf die Längsachse (28) hin weisende Führungsfläche (232) für das mindestens eine Feststellelement (172) bildet.

11. Medizinisches Schlaginstrument nach Anspruch 10, **dadurch gekennzeichnet, dass** der proximale Schaftabschnitt (22) den distalen Anschlag (30) umfasst mit einer in proximaler Richtung wirkenden ersten Anschlagfläche (44) und dass der Schlagkörper (26) eine erste, in distaler Richtung wirkende Schlagkörperanschlagfläche (234) umfasst, welche in der distalen Anschlagposition mit der ersten Anschlagfläche (44) zusammenwirkt,
wobei insbesondere der Schlagkörper (26) ein Anschlagelement (160) umfasst, welches die erste Schlagkörperanschlagfläche (234) definiert, wobei weiter insbesondere das Anschlagelement (160) eine Anschlagelementlängsachse (162) definiert und wobei die Anschlagelementlängsachse (162) quer, insbesondere senkrecht, zur Schaftlängsrichtung (20) verläuft.

12. Medizinisches Schlaginstrument nach einem der voranstehenden Ansprüche, **dadurch gekennzeichnet, dass** der Schlagkörper (26) mindestens eine Spülöffnung (244, 246) umfasst.

13. Medizinisches Schlaginstrument nach einem der voranstehenden Ansprüche, **dadurch gekennzeichnet, dass** das Schlaginstrument (12) eine Kopplungseinrichtung (58) umfasst zum temporären Koppeln mit einem medizinischen Objekt (14).

14. Medizinisches Schlaginstrument nach Anspruch 13, **dadurch gekennzeichnet, dass** die Kopplungseinrichtung (58) ein erstes Kopplungselement (64) umfasst, welches ausgebildet ist zum kraft- und/oder formschlüssigen in Eingriff Bringen mit einem korrespondierenden zweiten, vom medizinischen Objekt (14) umfassten Kopplungselement (66) in einer Kopplungsstellung.

15. Medizinisches Schlaginstrument nach Anspruch 13 oder 14, **dadurch gekennzeichnet, dass** die Kopplungseinrichtung (58) eine Sicherungseinrichtung (94) umfasst zum kraft- und/oder formschlüssigen Sichern eines medizinischen Objekts (14) am medizinischen Schlaginstrument (12) in der Kopplungsstellung.

## Claims

1. Medical impact instrument (12), in particular in the form of an impact hammer (16), comprising a shaft (18) defining a shaft longitudinal direction (20) as well as a proximal and a distal end (22, 24), and comprising an impact body (26) arranged on the shaft (18) and defining a longitudinal axis (28), wherein the impact body (26) in an impact position on the shaft (18) is movable, in particular displaceable, between a distal stop (30) defining a distal stop position of the impact body (26) and a proximal stop (32) defining a proximal stop position of the impact body (26) for transmitting an impact pulse to the distal end (24) of the shaft (18) in the distal or proximal direction, wherein the impact instrument (12) comprises a fixing device (34) for temporarily fixing the impact body (26) to the shaft (18) in at least one, in particular arbitrary, fixing position between the distal stop position and the proximal stop position, wherein the fixing device (34) is transferable from a fixing position, in which the impact body (26) is fixed to the shaft (18) in any one of the arbitrary fixing positions, into the impact position, in which the impact body (26) and the shaft (18) are movable relative to one another, and vice versa, **characterized in that** the impact body (26) in a basic position is fixed relative to the shaft (18) and adopts the at least one fixing position and **in that** the fixing device (34) comprises a restoring device (214) for automatically holding the fixing device (34) in the fixing position.

2. Medical impact instrument in accordance with Claim 1, **characterized in that** restoring device (214) comprises at least one restoring element (216),
wherein, in particular, the at least one restoring element (216) is configured in the form of a rubber-elastic or spring-elastic element (218) or in the form of a compression or tension spring (220).

3. Medical impact instrument in accordance with any one of the preceding Claims, **characterized in that** the fixing device (34) comprises at least one fixing element (172) for being brought into force-locking and/or positive-locking engagement both with the shaft (18) and with the impact body (26) in the at least one fixing position.

4. Medical impact instrument in accordance with Claim 3, **characterized in that** the at least one restoring element (216) is supported on a fixing element support face (188) of the at least one fixing element (172) on the one hand and on an impact body support face (156) of the impact body (26) on the other hand,
wherein, in particular, the fixing element support face (188) is configured pointing in the distal direction, in particular as an annular face, and wherein the impact body support face (156) is configured pointing in the proximal direction, in particular as an annular face (158).

5. Medical impact instrument in accordance with Claim 3 or 4, **characterized in that** the at least one fixing element (172)
a) is movable, namely displaceable, relative to the impact body (26) for transferring the fixing device (34) from the fixing position into the impact position
and/or
b) is of sleeve-shaped configuration
and/or
c) is movable relative to the impact body (26) in the distal direction for transferring the fixing device (34) from the locking position into the impact position
and/or
d) is closed on the proximal side.

6. Medical impact instrument in accordance with Claim 5, **characterized in that** the at least one fixing element (172) is configured in the form of a clamping element (196) and comprises at least one clamping arm (200) extending in the longitudinal direction, and **in that** a free end (222) of the at least one clamping arm (200) is configured to be movable, in particular pivotable, in the direction toward and away from the longitudinal axis (28) of the shaft (26).

7. Medical impact instrument in accordance with Claim 6, **characterized in that** the impact body (26) comprises a recess (150) surrounding the shaft (18) for accommodating the at least one clamping element (196).

8. Medical impact instrument in accordance with any one of the preceding Claims, **characterized in that** the impact body (26) has a longitudinal perforation (138) extending coaxially to the shaft longitudinal direction (20) and **in that** the shaft (18) is accommodated at least partially in the longitudinal perforation (138).

9. Medical impact instrument in accordance with Claim 8, **characterized in that** the shaft (18) comprises a distal shaft portion (36) and a proximal shaft portion (38), **in that** the proximal shaft portion (38) is accommodated in the longitudinal perforation (138), and **in that** the distal shaft portion (36) projects out of the longitudinal perforation (138) on the distal side.

10. Medical impact instrument in accordance with Claim 8 or 9, **characterized in that** the longitudinal perforation (138) of the impact body (26) commencing from its proximal end forms a guide face (232) pointing in the direction toward the longitudinal axis (28) for the at least one fixing element (172).

11. Medical impact instrument in accordance with Claim 10, **characterized in that** the proximal shaft portion (22) comprises the distal stop (30) with a first stop face (44) acting in the proximal direction, and **in that** the impact body (26) comprises a first impact body stop face (234) acting in the distal direction, which in the distal stop position interacts with the first stop face (44),
wherein, in particular, the impact body (26) comprises a stop element (160), which defines the first impact body stop face (234),
wherein, further in particular, the stop element (160) defines a stop element longitudinal axis (162) and wherein the stop element longitudinal axis (162) extends transversely, in particular perpendicularly, to the shaft longitudinal direction (20).

12. Medical impact instrument in accordance with any one of the preceding Claims, **characterized in that** the impact body (26) comprises at least one flush opening (244, 246).

13. Medical impact instrument in accordance with any one of the preceding Claims, **characterized in that** the impact instrument (12) comprises a coupling device (58) for temporarily coupling to a medical object (14).

14. Medical impact instrument in accordance with Claim 13, **characterized in that** the coupling device (58) comprises a first coupling element (64), which is configured to be brought into force-locking and/or positive-locking engagement with a corresponding second coupling element (66) comprised by the medical object (14) in a coupling position.

15. Medical impact instrument in accordance with Claim 13 or 14, **characterized in that** the coupling device (58) comprises a securing device (94) for securing a medical object (14) to the medical impact instrument (12) in the coupling position in a force-locking and/or positive-locking manner.

## Revendications

1. Instrument médical à percussion (12), en particulier sous la forme d'un marteau à percussion (16), comprenant un manche (18) définissant une direction longitudinale (20) du manche ainsi qu'une extrémité proximale et une extrémité distale (22, 24), et un corps de frappe (26) disposé sur le manche (18) et définissant un axe longitudinal (28), le corps de frappe (26) pouvant être déplacé, en particulier coulissé, dans une position de frappe sur le manche (18) entre une butée distale (30) définissant une position de butée distale du corps de frappe (26) et une butée proximale (32) définissant une position de butée proximale du corps de frappe (26), pour transmettre une impulsion de frappe à l'extrémité distale (24) du manche (18) dans la direction distale ou proximale, l'instrument à percussion (12) comprenant un dispositif de blocage (34) pour bloquer temporairement l'élément de frappe (26) sur le manche (18) dans au moins une position de blocage, en particulier une position quelconque, entre la position de butée distale et la position de butée proximale, le dispositif de blocage (34) pouvant être déplacé d'une position de fixation, dans laquelle le corps de frappe (26) est fixé sur le manche (18) dans l'une des positions de blocage quelconques, à la position de frappe, dans laquelle l'élément de frappe (26) et le manche (18) peuvent être déplacés l'un par rapport à l'autre, et inversement, **caractérisé en ce que** le corps de frappe (26) est bloqué dans une position de base par rapport au manche (18) et qu'il occupe au moins une position de blocage et **en ce que** le dispositif de blocage (34) comprend un dispositif de rappel (214) pour maintenir automatiquement le dispositif de blocage (34) dans la position de fixation.

2. Instrument médical à percussion selon la revendication 1, **caractérisé en ce que** le dispositif de rappel (214) comprend au moins un élément de rappel (216),
où, en particulier, le au moins un élément de rappel (216) étant réalisé sous la forme d'un élément élastique en caoutchouc ou à ressort (218) ou sous la forme d'un ressort de compression ou de traction (220).

3. Instrument médical à percussion selon l'une des revendications précédentes, **caractérisé en ce que** le dispositif de blocage (34) comprend au moins un élément de blocage (172) pour l'engagement par adhérence et/ou par complémentarité de forme à la fois avec le manche (18) et avec le corps de frappe (26) dans au moins une position de blocage.

4. Instrument médical à percussion selon la revendication 3, **caractérisé en ce que** l'au moins un élément de rappel (216) s'appuie d'une part sur une surface d'appui (188) de l'au moins un élément de blocage (172) et d'autre part sur une surface d'appui (156) du corps de frappe (26),
où, en particulier, la surface d'appui de l'élément de blocage (188) étant orientée dans le sens distal, en particulier sous la forme d'une surface annulaire, et la surface d'appui du corps de frappe (156) étant orientée dans le sens proximal, en particulier sous la forme d'une surface annulaire (158).

5. Instrument médical à percussion selon la revendication 3 ou 4, **caractérisé en ce que** le ou les éléments de blocage (172)
a) peut(vent) être déplacé(s), c'est-à-dire coulissé(s), par rapport au corps de frappe (26) pour faire passer le dispositif de blocage (34) de la position de fixation à la position de frappe,
et/ou
b) est ou sont en forme de manchon
et/ou
c) peut(vent) être déplacé(s) dans le sens distal par rapport au corps de frappe (26) pour faire passer le dispositif de blocage (34) de la position de fixation à la position de frappe
et/ou
d) est ou sont fermés du côté proximal.

6. Instrument médical à percussion selon la revendication 5, **caractérisé en ce que** le ou les éléments de blocage (172) est ou sont conçus sous la forme d'un élément de serrage (196) et comprend ou comprennent au moins un bras de serrage (200) s'étendant dans le sens longitudinal, et **en ce qu'**une extrémité libre (222) du ou des bras de serrage (200) est conçue de manière à pouvoir être déplacée, en particulier de manière pivotante, vers l'axe longitudinal (28) du manche (26) et à partir de celui-ci.

7. Instrument médical à percussion selon la revendication 6, **caractérisé en ce que** le corps de frappe (26) comprend un évidement (150) entourant le manche (18) pour recevoir le au moins un élément de serrage (196).

8. Instrument médical à percussion selon l'une des revendications précédentes, **caractérisé en ce que** le corps de frappe (26) présente une ouverture longitudinale (138) s'étendant coaxialement à la direction longitudinale du manche (20) et **en ce que** le manche (18) est logé au moins partiellement dans l'ouverture longitudinale (138).

9. Instrument médical à percussion selon la revendication 8, **caractérisé en ce que** le manche (18) comprend une partie distale (36) et une partie proximale (38), **en ce que** la partie proximale (38) est logée dans l'ouverture longitudinale (138) et **en ce que** la partie distale du manche (36) dépasse de l'ouverture longitudinale (138) du côté distal.

10. Instrument médical à percussion selon la revendication 8 ou 9, **caractérisé en ce que** l'ouverture longitudinale (138) du corps de frappe (26) forme, à partir de son extrémité proximale, une surface de guidage (232) orientée vers l'axe longitudinal (28) pour le ou les éléments de blocage (172).

11. Instrument médical à percussion selon la revendication 10, **caractérisé en ce que** la partie proximale du manche (22) comprend la butée distale (30) avec une première surface de butée (44) agissant dans la direction proximale et **en ce que** le corps de frappe (26) comprend une première surface de butée du corps de frappe (234) agissant dans le sens distal, qui coopère avec la première surface de butée (44) dans la position de butée distale,
où, en particulier, le corps de frappe (26) comprenant un élément de butée (160) qui définit la première surface de butée du corps de frappe (234),
où, en outre en particulier, l'élément de butée (160) définissant un axe longitudinal (162) de l'élément de butée et l'axe longitudinal (162) de l'élément de butée s'étendant transversalement, en particulier perpendiculairement, à la direction longitudinale (20) du manche.

12. Instrument médical à percussion selon l'une des revendications précédentes, **caractérisé en ce que** le corps de frappe (26) comprend au moins un orifice de rinçage (244, 246).

13. Instrument médical à percussion selon l'une des revendications précédentes, **caractérisé en ce que** l'instrument à percussion (12) comprend un dispositif de couplage (58) pour le couplage temporaire avec un objet médical (14).

14. Instrument médical à percussion selon la revendication 13, **caractérisé en ce que** le dispositif de couplage (58) comprend un premier élément de couplage (64) qui est conçu pour s'engager par adhérence et/ou par complémentarité de forme avec un deuxième élément de couplage (66) correspondant, compris par l'objet médical (14), dans une position de couplage.

15. Instrument médical à percussion selon la revendication 13 ou 14, **caractérisé en ce que** le dispositif de couplage (58) comprend un dispositif de sécurité (94) pour fixer par adhérence et/ou par complémentarité de forme un objet médical (14) sur l'instrument médical à percussion (12) dans la position de couplage.
